# EUROPEAN PATENT SPECIFICATION

(11) **EP 3 743 427 B1**
(45) Date of publication and mention of the grant of the patent: **19.10.2022**
(21) Application number: 19701346.9
(22) Date of filing: 22.01.2019
(51) Int. Cl.: C07D 471/14, A61K 51/04, C07B 59/00

(54) **NOVEL METHOD OF PREPARING AN IMAGING COMPOUND**
NEUARTIGES VERFAHREN ZUR HERSTELLUNG EINER BILDGEBUNGSVERBINDUNG
NOUVEAU PROCÉDÉ DE PRÉPARATION D'UN COMPOSÉ D'IMAGERIE

(30) Priority: 24.01.2018 EP 18153326
(43) Date of publication of application: 02.12.2020
(73) Proprietor: AC Immune SA, 1015 Lausanne (CH); Life Molecular Imaging Limited, Warwick CV34 6DA (GB)
(72) Inventor: ZERNA, Marion, 10405 Berlin (DE); BERNDT, Mathias, 12247 Berlin (DE); SCHIEFERSTEIN, Hanno, 65326 Aarbergen (DE); CASTILLO MELEAN, Johnny, 12439 Berlin (DE); KROTH, Heiko, 1024 Ecublens (CH); MOLETTE, Jérôme, 01280 Prévessin-Moëns (FR); DARMENCY, Vincent, 1169 Yens (CH); GABELLIERI, Emanuele, 1052 Le-Mont-sur-Lausanne (CH)
(74) Representative: Vossius & Partner Patentanwälte Rechtsanwälte mbB
(86) International application number: PCT/EP2019/051500
(87) International publication number: WO 2019/145294

(56) References cited:
- WO-A1-2015/052105
- WO-A1-2018/015549
- LUCA C. GOBBI ET AL: "Identification of Three Novel Radiotracers for Imaging Aggregated Tau in Alzheimer's Disease with Positron Emission Tomography", JOURNAL OF MEDICINAL CHEMISTRY, vol. 60, no. 17, 14 September 2017 (2017-09-14), pages 7350-7370, XP055556419, US ISSN: 0022-2623, DOI: 10.1021/acs.jmedchem.7b00632
- Luca C Gobbi ET AL: "Identification of Three Novel Radiotracers for Imaging Aggregated Tau in Alzheimer's Disease with Positron Emission Tomography - SUPPORTING INFORMATION", Journal of medicinal chemistry, vol. 60, no. 17, 14 September 2017 (2017-09-14), page 7350, XP055564091, United States

## Description

### Field of Invention

The present invention relates to a novel method of preparing a compound of the formula I that can be employed in the selective detection of disorders and abnormalities associated with Tau aggregates such as Alzheimer's disease (AD) and other tauopathies, for example, using Positron Emission Tomography (PET) imaging.

### Background

Alzheimer's disease is a neurological disorder primarily thought to be caused by amyloid plaques, an extracellular accumulation of abnormal deposit of amyloid-**beta (Aβ) aggregates** in the brain or in the eyes. The other major neuropathological hallmarks in AD are the intracellular neurofibrillary tangles (NFT) that originate by the aggregation of the hyperphosphorylated Tau (Tubulin associated unit) protein, phosphorylated Tau or pathological Tau and its conformers. AD shares this pathology with many neurodegenerative tauopathies, in particularly with specified types of frontotemporal dementia (FTD). In AD brain, Tau pathology (tauopathy) develops later than amyloid pathology, but it is still **discussed controversially if Aβ protein is the causative agent in A**D which constitutes the essence of the so-called amyloid cascade hypothesis (Hardy et al., Science 1992, 256, 184-185, and most recently, Musiek et al., Nature Neurosciences 2015, 18(6), 800-806, "Three dimensions of the amyloid hypothesis: time, space and 'wingmen‴).

WO 2018/015549 A1 relates to particular compounds that can be employed in the selective Tau detection of disorders and abnormalities associated with Tau aggregates such as Alzheimer's disease and other tauopathies using Positron Emission Tomography (PET) Imaging.

Presently, the only definite way to diagnose AD is to identify plaques and tangles in brain tissue by histological analysis of biopsy or autopsy materials after the death of the individual. Beside AD, Tau plays an important role in other (non-AD) neurodegenerative diseases. Such non-AD tauopathies include, for example, **supranuclear palsy (PSP), Pick's disease (PiD)** and corticobasal degeneration (CBD).

The compound of general formula **A** has been proposed as being useful in the selective detection of disorders and abnormalities associated with Tau aggregates such as **Alzheimer's disease (AD) and other tauopathies,** and certain methods of manufacturing this compound have been described in the prior art.

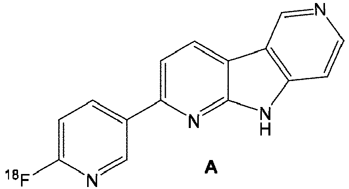

Gobbi et al. disclosed in WO 2015/052105 a method in which a compound of the formula **A** was obtained by reacting a precursor having a nitro group instead of ¹⁸F with [¹⁸F]fluoride in a microwave apparatus. After the synthesis step, the compound of the formula **A** was purified by a two-step procedure involving among others:
1) HPLC using a mobile phase of methanol and triethylamine; and
2) reformulation using a solid phase extraction cartridge for trapping the compound of the formula **A** and subsequent elution from the cartridge with ethanol diluted with saline (0.9% NaCl in water).

The method provided the compound of the formula **A** in a yield of 26.1%.

Gobbi et al. further disclosed the following two methods in J. Med. Chem. 2017, Volume 60, pages 7350 to 7370:
a) Microwave method: [¹⁸F]fluoride was trapped on a cation exchange cartridge and the activity was eluted with a Kryptofix^{®}/potassium carbonate mixture. The mixture was dried at elevated temperature after addition of acetonitrile. The vial with the dried [¹⁸F]fluoride mixture was then transferred to a microwave apparatus and 0.5 mg precursor molecule (unprotected nitro derivative) in 400 µL DMSO were added. The vial was irradiated by microwaves at 50 W for 240 seconds. The resulting solution was diluted and purified by preparative HPLC (C-18 column, acetonitrile/trimethylamine buffer at pH 7.2).
   The product peak was collected, diluted with water and passed through a C-18 Sep-Pak Plus cartridge. The cartridge was washed with water and the ¹⁸F-labelled product was eluted with ethanol and diluted with saline solution.
b) Thermal heating method: [¹⁸F]fluoride was trapped on a cation exchange cartridge and the activity was eluted with a Kryptofix^{®}/potassium carbonate mixture. The mixture was dried at elevated temperature after addition of acetonitrile. 0.5 mg precursor molecule (unprotected nitro derivative) in 400 µL DMSO were added and the solution was heated at 160 °C for 10 min. The resulting solution was diluted and purified by preparative HPLC (C-18 column, acetonitrile/trimethylamine buffer pH 7.2).
   The product peak was collected, diluted with water and passed through a C-18 Sep-Pak Plus cartridge. The cartridge was washed with water and the ¹⁸F-labelled product was eluted with ethanol and diluted with saline solution.

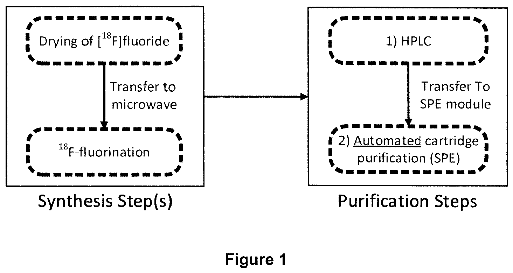

Thus, the methods for the preparation of the compound of the formula A in the aforementioned prior art have two purification/reformulation steps, wherein the method is only partially automated in the case of the microwave methods as illustrated in Figure 1.

The nitro precursor (and major side product) of the method described in the prior art has poor solubility especially in ethanol, acetonitrile and the acetonitrile/aqueous buffer mixtures used for purification (preparative HPLC), especially if aqueous buffer mixtures with neutral pH are used. Only 0.5 to 0.7 mg of the precursor were used in the examples of the prior art.

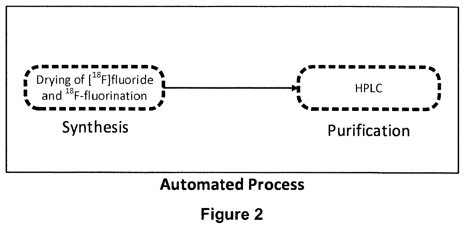

It is an object of the present invention to provide an improved method for preparing a compound of the formula I which is more cost and time efficient than prior art methods (as outlined in Figure 2). In addition, the yield of the method should be improved.

### Description of the Figures

- **Figure 1:**: Schematic overview of the prior art method chosen by Gobbi et al.
- **Figure 2:**: Schematic overview of the method of the present invention
- **Figure 3:**: Setup of the GE Tracerlab FX synthesizer
- **Figure 4:**: Setup of the IBA Synthera synthesizer

### Summary of the invention

The present invention is as defined in the claims. Thus, it relates to the following items 1 to 9:
1. A method of preparing a compound of the formula I 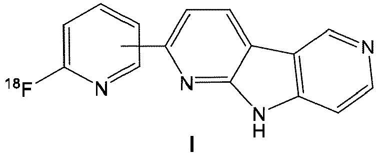 comprising the steps of
   A) reacting a compound of the formula II with a ¹⁸F fluorinating agent 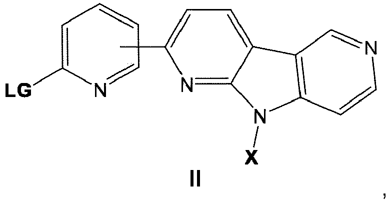 wherein **X** is H or **PG,**
      **LG** is a leaving group, and
      **PG** is an amine protecting group selected from carbobenzyloxy, (p-methoxybenzyl)oxycarbonyl, tert-butyloxycarbonyl, 9-fluorenylmethyloxycarbonyl, benzyl, p-methoxybenzyl, 3,4-dimethoxybenzyl, p-methoxyphenyl, triphenylmethyl, methoxyphenyl diphenylmethyl and dimethoxytrityl, and
   B) optionally, if **X** is **PG,** cleaving the protecting group **PG,** and
   C) subjecting the resultant compound of the formula I to high-performance liquid chromatography (HPLC) using a mobile phase comprising a mixture of ethanol and water.
2. The method according to item 1, wherein X is PG, step B is absent and the protecting group PG is cleaved in step A or wherein X is PG and the protecting group PG is cleaved in step B.
3. The method according to item 1 or 2, wherein the ratio of ethanol to water in the mobile phase is from 5/95 v/v to 80/20 v/v.
4. The method according to any of items 1 to 3, wherein the pH of the mobile phase is from 0 to 8, preferably 1 to 3.
5. The method according to any of items 1 to 4, wherein the mobile phase further comprises a buffer, which is preferably selected from alkali metal dihydrogen phosphates, di alkali metal hydrogen phosphates, tri alkali metal phosphates, alkali metal acetates, alkali metal formates, and mono/di/tri alkali metal citrates.
6. The method according to any one of items 1 to 5, wherein the high-performance liquid chromatography (HPLC) is conducted under a pressure of
   5* 10⁶ to 4* 10⁷ Pa (50 to 400 bar), preferably 5* 10⁶ to 25* 10⁶ Pa ( 50-250 bar).
7. The method according to any one of items 1 to 6, wherein the high-performance liquid chromatography (HPLC) results in a fraction comprising the compound of the formula I and this fraction is subjected to a step D) sterile filtration.
8. The method according to any one of items 1 to 7, wherein the method does not comprise solid phase extraction of the compound of the formula I after step C, preferably wherein the method does not comprise solid phase extraction of the compound of the formula I before or after step C.
9. The method according to any one of items 1 to 8, wherein the compound of the formula I is not subjected to chromatography after the high-performance liquid chromatography (HPLC) of step C, preferably wherein the compound of the formula I is not subjected to chromatography other than the high-performance liquid chromatography (HPLC) of step C.

### Definitions

**The term "alkyl" refers to a saturated straight or branched carbon chain, which, unless** specified otherwise, contain from 1 to 6 carbon atoms.

"Hal" or "halogen" represents F, Cl, Br and I. Preferably, "halogen" is, independently in each occurrence, selected from F, Cl and Br, more preferably, from F and Cl, even more preferably F.

**The term** "amine **protecting group" (PG)** as employed herein is any protecting group which is suitable for protecting an amine group during an envisaged chemical reaction. Examples of suitable protecting groups are well-known to a person skilled in the art. Suitable protecting groups are discussed, e.g., in the textbook Greene and Wuts, Protecting groups in Organic Synthesis, third edition, page 494-653. Protecting groups can be chosen from carbamates, amides, imides, N-alkyl amines, N-aryl amines, imines, enamines, boranes, N-P protecting groups, N-sulfenyl, N-sulfonyl and N-silyl. Specific preferred examples of protecting groups **(PG)** are carbobenzyloxy (Cbz), (p-methoxybenzyl)oxycarbonyl (Moz or MeOZ), tert-butyloxycarbonyl (BOC), 9-fluorenylmethyloxycarbonyl (FMOC), benzyl (Bn), p-methoxybenzyl (PMB), 3,4-dimethoxybenzyl (DMPM), p-methoxyphenyl (PMP), triphenylmethyl (Trityl), methoxyphenyl diphenylmethyl (MMT), or dimethoxytrityl (DMT). More preferred examples of the protecting group **PG** include tert-butyloxycarbonyl (BOC), dimethoxytrityl (DMT) and triphenylmethyl (Trityl). One more preferred example of the protecting group **PG** is tert-butyloxycarbonyl (BOC).

In the present invention, PG is an amine protecting group selected from carbobenzyloxy, (p-methoxybenzyl)oxycarbonyl, tert-butyloxycarbonyl, 9-fluorenylmethyloxycarbonyl, benzyl, p-methoxybenzyl, 3,4-dimethoxybenzyl, p-methoxyphenyl, triphenylmethyl, methoxyphenyl diphenylmethyl and dimethoxytrityl.

The term **"carbamate amine protecting group" refers to an amine protecting group containing** a *-CO-O group wherein the asterisk indicates the bond to the amine. Examples are carbobenzyloxy (Cbz), (p-methoxybenzyl)oxycarbonyl (Moz or MeOZ), tert-butyloxycarbonyl (BOC) and 9-fluorenylmethyloxycarbonyl (FMOC).

**The term "leaving group" (LG)** as employed herein is any leaving group and means an atom or group of atoms can be replaced by another atom or group of atoms. Examples are given e.g. in Synthesis (1982), p. 85-125, table 2, Carey and Sundberg, Organische Synthese, (1995), page 279-281, table 5.8; or Netscher, Recent Res. Dev. Org. Chem., 2003, 7, 71-83, scheme 1, 2, 10 and 15 and others). (Coenen, Fluorine-18 Labeling Methods: Features and Possibilities of Basic Reactions, (2006), in: Schubiger P.A., Friebe M., Lehmann L., (eds), PET-Chemistry - The Driving Force in Molecular Imaging. Springer, Berlin Heidelberg, pp.15-50, explicitly: scheme 4 pp. 25, scheme 5 pp 28, table 4 pp 30, Figure 7 pp 33). Preferably, the "leaving group" **(LG)** is selected from the group consisting of nitro, bromo, iodo, chloro, trialkyl ammonium, hydroxyl, boronic acid, iodonium, sulfonic ester. More preferably, the "leaving group" **(LG)** is nitro or trimethyl ammonium. It is to be understood that the compounds containing trialkyl ammonium or iodonium may further comprise an anion. Still more preferably, "leaving group" **(LG)** is nitro. Another more preferred "leaving group" **(LG)** is trimethyl ammonium.

The term "crown ether" as employed herein means chemical compounds that consist of a ring containing several ether groups. More specifically, the term "crown ether" refers to preferably monocyclic organic groups which may be substituted and contain from 8 to 16 carbon atoms and from 4 to 8 heteroatoms selected from N, O and S in the ring. Each of the one or more optional substituents may be independently selected from any organic group containing from 1 to 15 carbon atoms and optionally 1 to 6 heteroatoms selected from N, O and S. Preferred examples of the "crown ether" are optionally substituted monocyclic rings containing 10 to 14 carbon atoms and 5 to 7 heteroatoms selected from N, O and S in the ring. Examples of the "crown ether" are optionally substituted monocyclic rings containing 12 carbon atoms and 6 heteroatoms selected from N and O in the ring. Specific examples include 18-crown-6, dibenzo-18-crown-6, and diaza-18-crown-6.

The term "cryptand" as employed herein relates to a class of polycyclic compounds related to the crown ethers, having three chains attached at two nitrogen atoms. A well-known **"cryptand"** is 4,7,13,16,21,24-hexaoxa-1,10-diazabicyclo[8.8.8]hexacosane (Kryptofix^{®}).

Tau as used herein refers to a highly soluble microtubule binding protein mostly found in neurons and includes the major 6 isoforms, cleaved or truncated forms, and other modified forms such as arising from phosphorylation, glycosylation, glycation, prolyl isomerization, nitration, acetylation, polyamination, ubiquitination, sumoylation and oxidation. Pathologic Tau or Tau aggregates (Neurofibrillary Tangles, NFTs) as used herein refer to insoluble aggregates of the hyperphosphorylated Tau protein containing paired helical filaments and straight filaments. Their presence is a hallmark of AD and other diseases known as tauopathies.

The tau gene contains 16 exons with the major tau protein isoforms being encoded by 11 of them The alternative splicing of exon 10 generates tau isoforms with either three (exon 10 missing) or four (exon 10 present) repeat domains, known as 3R and 4R tau, respectively (A. Andreadis et al., Biochemistry 31, (1992) 10626 - 10633; M. Tolnay et al., IUBMB Life, 55(6): 299-305, 2003). In Alzheimer's disease, the ratio of 3R and 4R isoforms is similar. In contrast thereto, in some tauopathies one of the two isoforms is predominantly present. Herein, the term "3R tauopathy" refers to tauopathies (such as **Pick's disease (PiD))** in which the 3R isoform is predominantly present. Herein, the term "4R tauopathy" refers to tauopathies (such as progressive supranuclear palsy (PSP) and corticobasal degeneration (CBD)) in which the 4R isoform is predominantly present.

As used hereinafter in the description of the invention and in the claims, the term **"pharmaceutically acceptable salt" relates** to non-toxic derivatives of the disclosed compounds wherein the parent compound is modified by making salts of inorganic and organic acids thereof. Inorganic acids include, but are not limited to, acids such as hydrochloric, nitric or sulfuric acid. Organic acids include, but are not limited to, carboxylic and sulfonic acids such as aliphatic, cycloaliphatic, aromatic, araliphatic and heterocyclic acids. The pharmaceutically acceptable salts can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two. Lists of suitable salts can be found in Remington's Pharmaceutical Sciences, 18th ed., Mack Publishing Company, Easton, PA, 1990, p. 1445.

"Pharmaceutically acceptable" or "diagnostically acceptable" are defined as referring to those compounds, materials, compositions, and/or dosage forms which are, within the scope of sound medical judgment, suitable for use in contact with the tissues of human beings and animals without excessive toxicity, irritation, allergic response, or other problem or complication commensurate with a reasonable benefit/risk ratio.

The patients or subjects are typically animals, particularly mammals, more particularly humans.

"Chromatography" or "liquid chromatography" means a method for the separation of a mixture of compounds. The mixture is dissolved in a fluid and transported via a "mobile phase" through a "stationary phase". The separation is based on the interaction of the compounds in the mobile phase with the stationary phases. Such different interactions result in differential retention on the stationary phase and thus affect the separation. Chromatography may be preparative or analytical. The purpose of preparative chromatography is to separate the components of a mixture, and is thus a form of purification. Analytical chromatography is done with a small sample of material and is used to measure the proportions of compounds in a mixture.

"High-performance liquid chromatography (HPLC)" is a form of liquid chromatography to separate compounds by using very small particles of the stationary phase (≤10 µm) and applying sufficiently higher pressures. An HPLC system typically consists of a reservoir of mobile phase(s), a pump, an injector, a separation column (containing the stationary phase), and detectors. For separation of radioactive compounds, suitable HPLC systems are equipped with a radioactivity detector. Optionally, the HPLC system has additional detectors, such as for example UV, photo diode array, refractive index, conductivity, fluorescence, mass spectrometer.

"Solid phase extraction (SPE)" is a sample preparation and/or purification process with two or more separate steps. First, the compounds are dissolved or suspended in a liquid mixture of solvents and the liquid sample is passed through a stationary (solid) phase. Some compounds are retained on the stationary phase while others pass through. In the second step, the retained compounds are eluted with a suitable solvent. Optionally, the stationary phase is washed with another solution before the elution step. In contrast to the HPLC technique, the used particle size is much bigger (e.g. ≥ 25 µm compared to HPLC with a typical particle size of ≤ 10 µm) and therefore, the applied pressure is much lower (for HPLC the pressure is typically > 50 bar).

"Solid phase extraction cartridge (SPE cartridge)" is a syringe or container (e.g. Sep Pak^{®}) prefilled with the stationary phase for SPE.

"Sterile filtration" is a method for sterilization of a solution by filtration via a microfilter. A microfilter is a filter having, e.g., a pore size of about 0.25 µm or less, preferably about 20 nm to about 0.22 µm, which is usually used to remove microorganisms. Membrane filters used in microfiltration in production processes are commonly made from materials such as mixed cellulose ester, polytetrafluorethylene (PTFE), polyvinylidene fluoride (PVDF) or polyethersulfone (PES).

"Automated" used herein, means the conduction of synthesis and or purification steps by a suitable apparatus (synthesizer).

The term "radioscavenger" refers to a compound that decreases the rate of decomposition due to radiolysis. Preferred radioscavengers include ascorbic acid and salts thereof and gentisic acid and salts thereof. More preferred radioscavengers are ascorbic acid, sodium ascorbate and mixtures thereof.

Suitable "synthesizers" for ¹⁸F-radiolabeling are well known to the person skilled in the art including but not limited to IBA Synthera, GE Fastlab, GE Tracerlab MX, GE Tracerlab FX, GE Tracerlab FX, Trasis AllinOne, ORA Neptis Perform, ORA Neptis Mosaic, ORA Neptis Plug, Scintomics GPR, Synthera, Comecer Taddeo, Raytest Synchrom, Sofie Elixys, Eckert&Ziegler Modular Lab, Sumitomo Heavy Industries F100 F200 F300, Siemens Explora.

"Radiochemical purity" means that a proportion of the total activity of the radionuclide present in its stated chemical form. Typically, the radiochemical purity is determined by thin-layer-chromatography or HPLC.

The preferred definitions given in the "Definitions"-section apply to all of the embodiments described herein unless stated otherwise.

### The methods of the present invention

The present invention is directed to a method for preparing a compound of the formula I

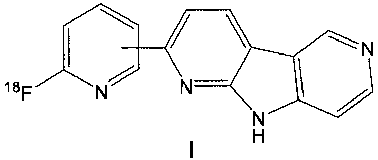

This method comprises the steps of:
A) reacting a compound of the formula II with a ¹⁸F fluorinating agent 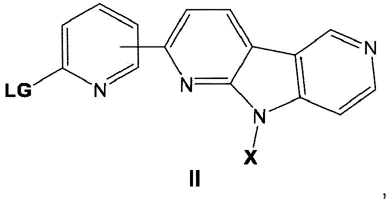 wherein **X** is H or **PG,**
   **LG** is a leaving group, and
   **PG** is an amine protecting group selected from carbobenzyloxy, (p-methoxybenzyl)oxycarbonyl, tert-butyloxycarbonyl, 9-fluorenylmethyloxycarbonyl, benzyl, p-methoxybenzyl, 3,4-dimethoxybenzyl, p-methoxyphenyl, triphenylmethyl, methoxyphenyl diphenylmethyl and dimethoxytrityl, and
B) optionally, if **X** is **PG,** cleaving the protecting group **PG,** and
C) subjecting the resultant compound of the formula I to high-performance liquid chromatography (HPLC) using a mobile phase comprising a mixture of ethanol and water.

Preferred compounds of the formula I are selected from the group consisting of

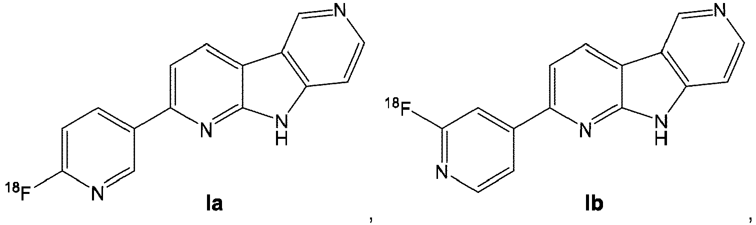

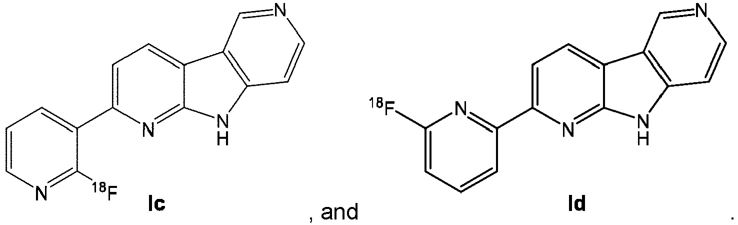

A more preferred compound of the formula **I** is

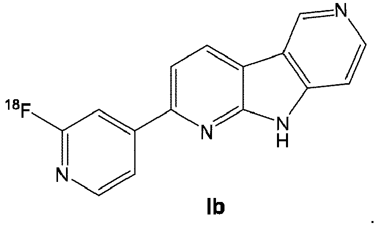

Preferred compounds of the formula **II** are selected from the group consisting of

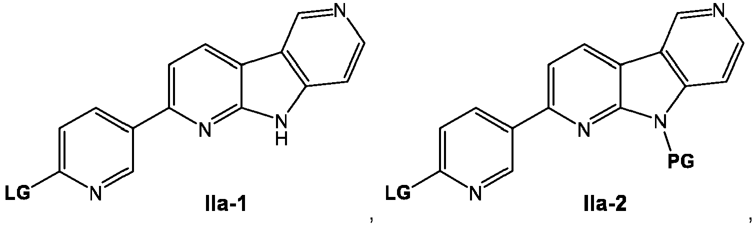

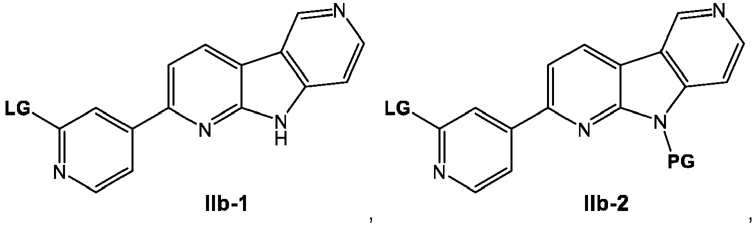

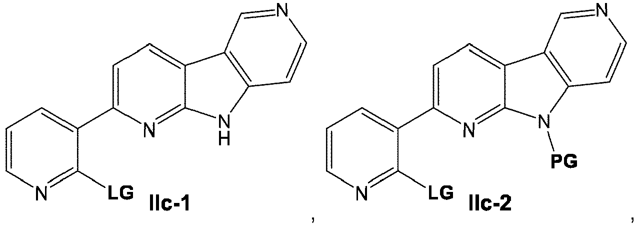

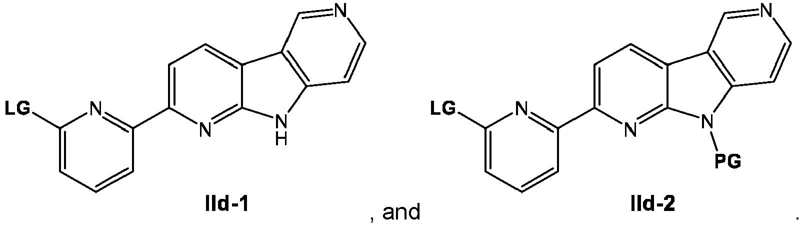

More preferred compounds of the formula II are selected from the group consisting of

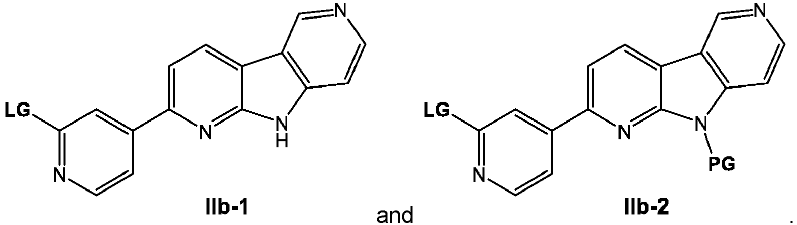

In these compounds PG and LG are as defined in the "Definitions"-section.

Even more preferred compounds of the formula II are selected from the group consisting of 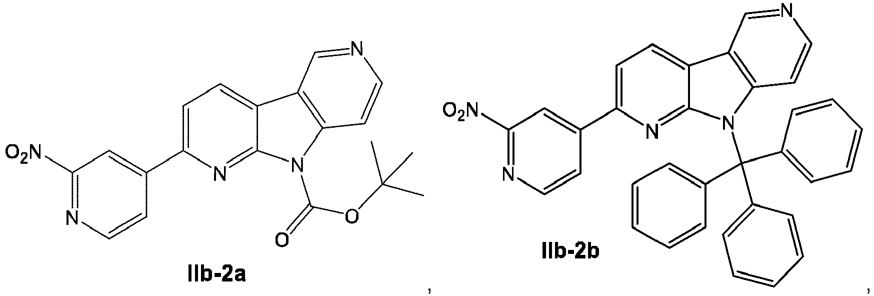 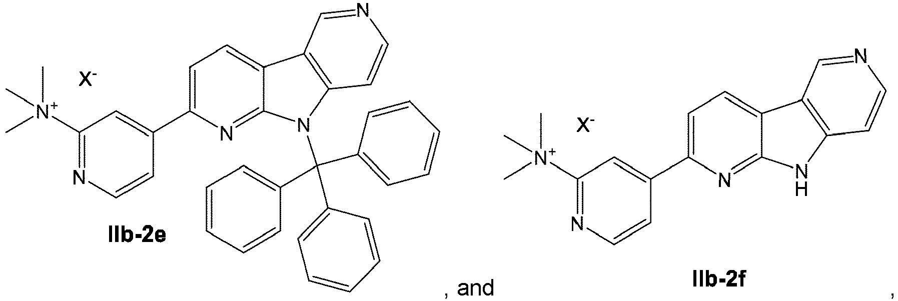 with X⁻ being a counter ion such as a counter ion selected from the group consisting of halogen, CF₃SO₃⁻, and CF₃CO₂⁻.

### Step A

Step A comprises reacting a compound of the formula II with a ¹⁸F fluorinating agent 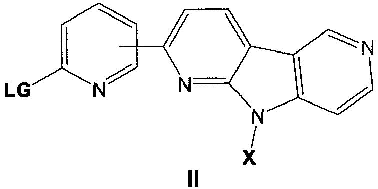 wherein
**X** is H or **PG,**
**LG** is a leaving group, and
**PG** is an amine protecting group selected from carbobenzyloxy, (p-methoxybenzyl)oxycarbonyl, tert-butyloxycarbonyl, 9-fluorenylmethyloxycarbonyl, benzyl, p-methoxybenzyl, 3,4-dimethoxybenzyl, p-methoxyphenyl, triphenylmethyl, methoxyphenyl diphenylmethyl and dimethoxytrityl.

If **X** is H a compound having the formula I will result. If **X** is **PG** an intermediate compound having the formula III will be obtained.

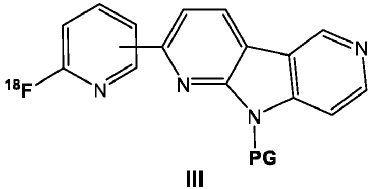

¹⁸F fluorinating agents are well known to the person skilled in the art. Any suitable ¹⁸F-fluorinating agent can be employed. Typical examples include H¹⁸F, alkali or alkaline earth ¹⁸F-fluorides (e.g., K¹⁸F, Rb¹⁸F, Cs¹⁸F, and Na¹⁸F). Optionally, the ¹⁸F-fluorinating agent can be used in combination with a chelating agent such as a cryptand (e.g.: 4,7,13,16,21,24-hexaoxa-1,10-diazabicyclo[8.8.8]-hexacosane - Kryptofix^{®}) or a crown ether (e.g.: 18-crown-6). Alternatively, the ¹⁸F-fluorinating agent can be a tetraalkyl ammonium salt of ¹⁸F or a tetraalkyl phosphonium salt of ¹⁸F; e.g., tetra(C₁₋₆ alkyl)ammonium salt of ¹⁸F or a tetra(C₁₋₆ alkyl)phosphonium salt of ¹⁸F. Examples thereof include tetrabutyl ammonium [¹⁸F]fluoride and tetrabutyl phosphonium [¹⁸F]fluoride. Preferably, the ¹⁸F-fluorinating agent is K¹⁸F, H¹⁸F, Cs¹⁸F, Na¹⁸F or tetrabutyl ammonium [¹⁸F]fluoride. In an even more preferred embodiment, the ¹⁸F-fluorinating agent is K¹⁸F. In another more preferred embodiment, the ¹⁸F-fluorinating agent is tetrabutyl ammonium [¹⁸F]fluoride.

The ¹⁸F-fluorination is typically carried out in a solvent which is preferably selected from acetonitrile, dimethylsulfoxide, dimethylformamide, dimethylacetamide, amyl alcohol, *tert-*butyl alcohol, or mixtures thereof, preferably the solvent contains or is acetonitrile or DMSO. But also other solvents can be used which are well known to a person skilled in the art. The solvent may further comprise water and/or other alcohols, such as C₁₋₁₀ linear, branched or cyclic alkanols, as a co-solvent. In one preferred embodiment the solvent for carrying out the ¹⁸F radiolabeling contains dimethyl sulfoxide. In another preferred embodiment the solvent for carrying out the ¹⁸F radiolabeling contains acetonitrile. In one preferred embodiment the solvent for carrying out the ¹⁸F radiolabeling is dimethyl sulfoxide. In another preferred embodiment the solvent for carrying out the ¹⁸F radiolabeling is acetonitrile.

The ¹⁸F-fluorination is typically conducted for at most about 60 minutes. Preferred reaction times are at most about 30 minutes. Further preferred reaction times are at most about 15 min. Typical reaction times are about 1-15 min, preferably 5-15 min, more preferably 10-15 min.

The ¹⁸F-fluorination is typically carried out at a temperature of about 60 to about 200 °C under conventional or microwave-supported heating. In a preferred embodiment, the ¹⁸F-fluorination is carried out at about 100 to about 180 °C. In a more preferred embodiment, the ¹⁸F-fluorination is carried out at about 100 to about 160 °C. Preferably, the ¹⁸F-fluorination is carried out under conventional heating. Conventional heating is understood to be any heating without the use of microwaves.

The amount of starting material is not particularly limited. For example, about 0.5 to about 50 µmol of a compound of the formula **II** can be used for the production of the compound of the formula **I** in one batch. In a preferred embodiment, about 2 to about 25 µmol of a compound of the formula **II** are used. In a more preferred embodiment, about 2.5 to about 15 µmol of a compound of the formula **II** are used. In one embodiment at least about 2 µmol of a compound of the formula **II** are used. In a preferred embodiment, at least about 2.5 µmol of a compound of the formula **II** are used. In a more preferred embodiment, at least about 3 µmol of a compound of the formula **II** are used.

Typically, about 0.5 to about 10 mg of the formula **II** can be used for the production of the compound of the formula **I** in one batch. In a preferred embodiment, about 0.5 to about 5 mg of a compound of the formula **II** are used. In a more preferred embodiment, about 1 to about 3 mg of a compound of the formula **II** are used.

If **X** is **PG** an intermediate compound having the formula **III** will be obtained. The protecting group **PG** can either be cleaved during the step A or in an optional subsequent step B.

Preferred compounds of the formula **III** are selected from the group comprising

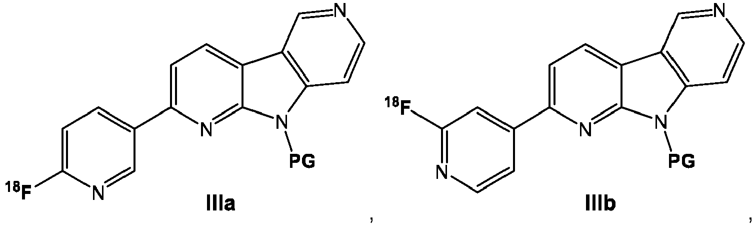

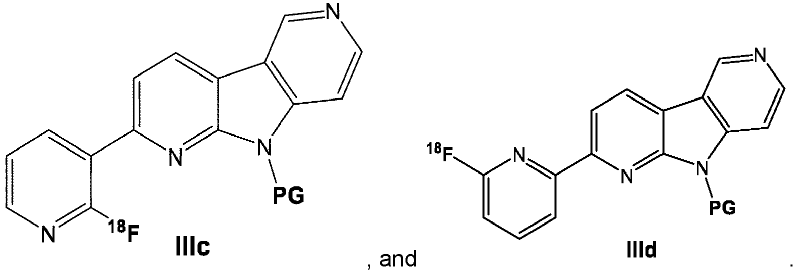

In these compounds **PG** is as defined in the "Definitions"-section.

### Step B

**Step B** is an optional step which comprises the cleavage of a protecting group **PG** from a compound of the formula **III** to obtain a compound of the formula **I.** As will be apparent to a skilled person, this step is not applicable if step A is conducted with a compound of the formula **II** in which **X** is hydrogen or if the protecting group **PG** is already cleaved in step A.

Reaction conditions for the cleavage of a large variety of protecting groups are well-known to a person skilled in the art and may be chosen from but are not limited to those described in the textbook by Greene and Wuts, Protecting groups in Organic Synthesis, third edition, page 494-653, and the textbook by P. J. Kocienski, Protecting Groups, 3rd Edition 2003.

The conditions which are employed in step B will depend on the protecting group which is to be cleaved and are thus not particularly limited.

Possible reaction conditions include i) heating at about 60 to about 160 °C, ii) addition of an acid and heating at about 0 °C to about 160 °C; or iii) addition of a base and heating at about 0 °C to about 160 °C.

Preferred acids are hydrochloric acid, sulfuric acid, and phosphoric acid. One preferred acid is sulfuric acid. Another preferred acid is phosphoric acid. Another preferred acid is hydrochloric acid. Preferred bases are sodium hydroxide, potassium hydroxide.

A preferred reaction condition is addition of an acid and heating at about 25 °C to 160 °C, preferably 25 °C to 120 °C, more preferably 90-120 °C. Preferably, the reaction mixture is heated for about 1 to about 20 min, more preferably for about 5 to about 15 min.

If desired, Steps A and B can be performed in the same or different reaction vessels. Preferably, Steps A and B are performed in the same reaction vessel.

If desired, the solution obtained after Step B can be used as such in Step C. Alternatively, the composition of the solution can be adapted, so that it is more appropriate for conducting HPLC. For instance, a buffer or diluent can be added prior to Step C.

Preferred diluents are ethanol, water; or a combination thereof.

In addition, the pH of the solution can be adapted. In a preferred embodiment, the pH is adjusted to about 6 or less, more preferably about 4 or less or even more preferably about 3 or less before Step C. In a preferred embodiment, the pH is adjusted to about 0 to about 6, more preferably about 0 to about 4, even more preferably about 1 to about 3, before Step C.

### Step C

Step C is a step in which the compound of the formula I obtained in Step A or, if employed, Step B, is subjected to HPLC using a mobile phase comprising a mixture of ethanol and water.

In the prior art, it was assumed that it is necessary to employ two chromatographic steps in order to arrive at an injectable tracer, i.e. first purification by HPLC and then reformulation by SPE of the compound of the formula I. The present inventors have surprisingly found that if a mixture of ethanol and water is used as a mobile phase subsequent trapping of the compound via solid phase extraction can be omitted. Therefore, it is possible to use a single chromatographic step for purification and formulation. This is particularly important in the present case of radiofluorination, as ¹⁸F has a half-life of only about 110 minutes, so that the speed of the method to arrive at an injectable formulation of compound I is of utmost importance.

Since the presently claimed method is faster and less complex than prior methods, the compound of formula I can be obtained in higher non-decay corrected yield.

The choice of the mixture of ethanol and water as a mobile phase has the additional advantage that these two components are diagnostically acceptable, so that (unlike methanol, acetonitrile, triethylamine and trimethylamine which are employed in the prior art) they can remain in the composition which is administered to the patient. Therefore, the choice of ethanol and water as the mobile phase significantly reduces the time and costs required in the preparation of the compound of the formula I and/or gives higher yields and higher radiochemical purity than the methods of the prior art.

The method of the present invention preferably does not comprise solid phase extraction of the compound of the formula I after step C, more preferably the method of the present invention does not comprise solid phase extraction of the compound of the formula I before or after step C.

The compound of the formula I is preferably not subjected to chromatography after the high-performance liquid chromatography (HPLC) of step C, more preferably the compound of the formula I is not subjected to chromatography other than the high-performance liquid chromatography (HPLC) of step C.

The mobile phase used in the HPLC method comprises ethanol and water. Furthermore, an acid, a base, a buffer, a salt and/or a radioscavenger and optionally mixtures thereof can be contained.

The ratio of ethanol to water is not particularly limited but is preferably about 5/95 v/v to about 80/20 v/v, more preferably about 5/95 v/v to about 50/50 v/v, even more preferably about 5/95 v/v to about 20/80 v/v.

The pH of the mobile phase is not restricted, but it is preferably from about 0 to about 8, preferably about 0 to about 6, more preferably about 1 to about 5, even more preferably about 1 to about 3, and even more preferably about 2.2 to about 2.8.

Possible buffers may include salts which can be selected from alkali metal dihydrogen phosphates, di alkali metal hydrogen phosphates, tri alkali metal phosphates, alkali metal acetates, alkali earth metal acetates, alkali earth metal formates, mono/di/tri alkali metal citrate, with the preferred alkali and alkali earth metals being sodium and potassium.

Possible bases can be sodium hydroxide and/or potassium hydroxide.

If desired, the pH of the mobile phase can be adjusted using an inorganic or organic acid. Examples of inorganic acids include ascorbic acid, citric acid, and acetic acid. Examples of organic acids include hydrochloric acid, sulfuric acid, and phosphoric acid, preferably phosphoric acid.

Radioscavengers are compounds which decrease the decomposition of the compound of formula I by radiolysis. Examples include ascorbic acid and ascorbic acid salts, gentisic acid and gentisic acid salts. Further examples include citric acid and citric acid salts. More preferred radioscavengers are ascorbic acid, sodium ascorbate and mixtures thereof.

Preferably, the mobile phase comprises about 50 to about 500 mM buffer (e.g., alkali dihydrogen phosphate), with a pH of about 1 to about 3, more preferably about 50 to about 250 mM buffer (e.g., alkali dihydrogen phosphate), with a pH of about 1 to about 3, even more preferably about 50 to about 150 mM buffer (e.g., alkali dihydrogen phosphate), with a pH of about 1 to about 3.

A preferred mobile phase comprises about 5 to about 20 % v/v ethanol, about 95 to about 80 % v/v water, about 50 to about 150 mM buffer (e.g., alkali dihydrogen phosphate), with a pH of about 1 to about 3, and optionally a radioscavenger. A more preferred mobile phase comprises about 5 to about 20 % v/v ethanol, about 95 to about 80 % v/v water, about 50 to about 150 mM buffer (e.g., alkali dihydrogen phosphate), with a pH of about 2.2 to about 2.8, and optionally a radioscavenger.

Stationary phases for use in HPLC methods are well-known and can be appropriately chosen by a skilled person. In a preferred embodiment, the stationary phase is a "reversed phase" (RP) stationary phase.

Examples of RP-HPLC stationary phases include C18, C8, phenyl, cyano (e.g. cyanopropyl), pentafluorophenyl, amino (e.g. aminopropyl), amide (e.g. C₁₀₋₂₄-alkanoic-aminopropyl), phenyl hexyl functionalized resins or mixed phase resins.

In one embodiment, the particle size of the HPLC stationary phase is about 1.6 to about 15 µm. In a preferred embodiment, the particle size of the HPLC stationary phase is about 5 to about 10 µm. In another embodiment, the particle size of the HPLC stationary phase is about 10 µm.

Typically, the HPLC column has a diameter of about 2.0 to about 50 mm and a length of about 50 to about 300 mm. In a preferred embodiment, the HPLC column has a diameter of about 4.6 to about 20 mm and a length of about 150 to about 250 mm. In a more preferred embodiment, the HPLC column has a dimension of 10 x 250 mm.

The flow rate employed in the high-performance liquid chromatography is not restricted and can be from about 1 to about 20 mL/min, more typically from about 2 to about 15 mL/min, even more typically from about 2 to about 7 mL/min.

The pressure employed in the high-performance liquid chromatography is not particularly limited and can be in the range of about 50 to about 400 bar, typically from about 50 to about 250 bar, more typically from about 50 to 200 bar.

In one embodiment, about 1 to about 500 GBq [¹⁸F]fluoride are used for the production of the compound of the formula I. In a preferred embodiment, about 50 to about 500 GBq [¹⁸F]fluoride are used for the production of the compound of the formula I. In a more preferred embodiment, about 100 to about 500 GBq [¹⁸F]fluoride are used for the production of the compound of the formula I. In an even more preferred embodiment, about 200 to about 500 GBq [¹⁸F]fluoride are used for the production of the compound of the formula I.

In one embodiment, about 10 GBq or more [¹⁸F]fluoride are used for the production of the compound of the formula I. In a preferred embodiment, about 50 GBq or more [¹⁸F]fluoride are used for the production of the compound of the formula I. In a more preferred embodiment, about 100 GBq or more [¹⁸F]fluoride are used for the production of the compound of the formula I. In an even more preferred embodiment, about 200 GBq or more [¹⁸F]fluoride are used for the production of the compound of the formula I.

In one embodiment, about 10 GBq or more of radiolabeled compound of the formula I are obtained. In a preferred embodiment, about 20 GBq or more of radiolabeled compound of the formula I are obtained. In a more preferred embodiment, about 50 GBq or more of radiolabeled compound of the formula I are obtained. In an even more preferred embodiment, about 100 GBq or more of radiolabeled compound of the formula I are obtained.

In one embodiment, the compound of the formula I is obtained with a radiochemical purity of at least about 90%. In a preferred embodiment, the compound of the formula I is obtained with a radiochemical purity of at least about 93%. In a preferred embodiment, the compound of the formula I is obtained with a radiochemical purity of at least about 95%. In a more preferred embodiment, the compound of the formula I is obtained with a radiochemical purity of at least about 98%.

### Optional steps

Since ethanol and water are comprised as a mixture in the mobile phase, the HPLC fraction comprising the compound of the formula I can be directly used as an injectable formulation, if desired. Alternatively, the HPLC fraction comprising the compound of the formula I can mixed with further diagnostically acceptable components such as a diagnostically acceptable carrier, diluent, adjuvant or excipient to prepare an injectable formulation of the compound of the formula I.

If desired Step D, sterile filtration can be conducted after Step C. If further components are added, the sterile filtration can be conducted before or after their addition.

In one embodiment, Step A, optional Step B and Step C are performed using an automated synthesis device. Examples of such automated synthesis devices include, but are not limited, to IBA Synthera, GE Fastlab, GE Tracerlab MX, GE Tracerlab FX, GE Tracerlab FX, Trasis AllinOne, ORA Neptis Perform, ORA Neptis Mosaic, ORA Neptis Plug, Scintomics GPR, Synthera, Comecer Taddeo, Raytest Synchrom, Sofie Elixys, Eckert&Ziegler Modular Lab, Sumitomo Heavy Industries F100 F200 F300, and Siemens Explora.

One preferred method comprises the steps of:
A) reacting a compound of the formula **II,** wherein **LG** = Nitro and **PG** = Boc with a [¹⁸F]-fluorinating agent, preferably selected from K¹⁸F and tetrabutylammonium [¹⁸F]fluoride, in DMSO at about 100 to about 180 °C, preferably about 120 to about 180 °C, more preferably about 140 to about 160 °C, wherein the Boc protecting group is cleaved under the radiolabeling conditions,
C) HPLC purification of the compound of the formula **I** using an ethanol/sodium dihydrogen phosphate buffer mixture (about 5 to about 20% EtOH), wherein the buffer has a pH of about 1 to about 3, preferably about 2.2 to about 2.8 and
D) if desired, mixing the HPLC fraction comprising the compound of the formula I with further diagnostically acceptable components of the formulation intended for administration to a patient. If desired the HPLC fraction is passed through a sterile filter before or after mixing with further diagnostically acceptable components of the formulation.

Another preferred method comprises the steps of:
A) reacting a compound of the formula **II,** wherein **LG** = trimethyl ammonium, and **PG** = Trityl with a [¹⁸F]-fluorinating agent, preferably selected from K¹⁸F and tetrabutylammonium [¹⁸F]fluoride, in DMSO or acetonitrile at about 80 to about 180 °C, preferably about 100 to about 180 °C,
B) addition of phosphoric acid and heating at about 90 to about 120 °C for about 1 to about 15 min,
C) HPLC purification of the compound of the formula I using an ethanol/sodium dihydrogen phosphate buffer mixture (about 5 to about 20% EtOH), wherein the buffer has a pH of about 1 to about 3, preferably about 2.2 to about 2.8 and,
D) if desired, mixing the HPLC fraction comprising the compound of the formula I with further diagnostically acceptable components of the formulation intended for administration to a patient. If desired the HPLC fraction is passed through a sterile filter before or after mixing with further diagnostically acceptable components of the formulation.

Unless otherwise specified, every hydrogen in the compounds of the formulas **I, II** or **III** can be ¹H or ²H (deuterium).

The method of the present invention can provide a diagnostic composition comprising a compound of the formula **I.** Due to the speed and the reduced complexity of the instant method, the amount of ¹⁸F-labeled compound I can be higher than in conventional methods. This method also provides compound of the formula **I** with a high radiochemical purity (e.g. at least about 90%, preferably at least about 93%, more preferably at least about 95%, even more preferably at least about 98%) at high radioactivity levels (e.g. at least about 20 GBq of compound of formula **I,** preferably at least about 50 GBq of compound of formula **I,** more preferably at least about 100 GBq of compound of formula **I).**

The instant diagnostic compositions are suitable for use in diagnostics. They are particularly suitable for diagnosing a disorder associated with Tau aggregates or imaging of Tau aggregates, particularly for imaging Tau aggregates using positron emission tomography (PET).

### Diagnostic compositions (disclosed, but not claimed)

A "diagnostic composition" is defined as a composition comprising a compound of the formula **I.** For *in vivo* applications the diagnostic composition should be in a form suitable for administration to mammals such as humans. Preferably a diagnostic composition further comprises a physiologically acceptable carrier, diluent, adjuvant or excipient. Administration to a patient is preferably carried out by injection of the composition as a solution. Such a composition may optionally contain further ingredients such as solvents, buffers; diagnostically acceptable solubilizers; and diagnostically acceptable stabilizers or antioxidants.

Diagnostically acceptable excipients are well known in the pharmaceutical art, and are described, for example, in Remington's Pharmaceutical Sciences, 15th Ed., Mack Publishing Co., New Jersey (1975). The diagnostic excipient can be selected with regard to standard pharmaceutical practice. The excipient must be acceptable in the sense of being not deleterious to the recipient thereof.

Preferably, the diagnostic composition comprises about 1 % v/v to about 20 % v/v ethanol, based on the total amount of ethanol and water. More preferably, the diagnostic composition comprises about 1 % v/v to about 15 % v/v ethanol, based on the total amount of ethanol and water. Even more preferably, the diagnostic composition comprises about 5 % v/v to about 10 % v/v ethanol, based on the total amount of ethanol and water. In addition to the above components the diagnostic composition comprises water. The amount of water is chosen, so that the total amount of the composition is 100 %.

The compounds of the formula I are to be administered via injection. Examples of such administration include one or more of: intravenously, intraarterially, intraperitoneally, intrathecally, intraventricularly, intraurethrally, intrasternally, intracranially, intramuscularly or subcutaneously administering the compounds; and/or by using infusion techniques. For parenteral administration, the compounds are best used in the form of a sterile solution which may contain other excipients. The solutions should be suitably buffered (preferably to a pH of from 3 to 9, more preferably from 4.0 to 8.5), if necessary. The preparation of suitable parenteral formulations under sterile conditions is readily accomplished by standard pharmaceutical techniques well known to those skilled in the art.

The diagnostic compositions can be formulated in a manner known per se to the skilled person as described, for example, in Remington's Pharmaceutical Sciences, 15th Ed., Mack Publishing Co., New Jersey (1975).

The dose of the detectably labeled compounds of the formula I may vary depending on the exact compound to be administered, the weight of the patient, size and type of the sample, and other variables as would be apparent to a physician skilled in the art. Generally, the mass could preferably lie in the range of about 0.001 µg to about 100 µg per patient dose, preferably about 0.01 µg to about 50 µg per patient dose. The radioactive dose can be, e.g., about 100 to about 600 MBq, more preferably about 150 to about 450 MBq per injection, even more preferably about 150 to about 200 MBq.

In particular, in one embodiment diseases or disorders that can be detected and monitored with the detectably labeled compounds of the formula I are diseases or conditions associated Tau proteins aggregates, such as 3R or 4R tauopathies.

The diseases or conditions that can be detected and monitored with the detectably labeled compounds obtained by the method of the present invention include neurodegenerative disorders such as tauopathies. Examples of diseases and conditions which can be detected and monitored are caused by or associated with the formation of neurofibrillary lesions. This is the predominant brain pathology in tauopathy. The diseases and conditions comprise a heterogeneous group of neurodegenerative diseases or conditions including diseases or conditions which show co-existence of Tau and amyloid pathologies. Examples of diseases involving Tau aggregates are generally listed as tauopathies and these include, but are not limited to, Alzheimer's disease (AD), Creutzfeldt-Jacob disease, dementia pugilistica, Down's Syndrome, Gerstmann-Straussler-Scheinker disease, inclusion-body myositis, prion protein cerebral amyloid angiopathy, traumatic brain injury, amyotrophic lateral sclerosis, Parkinsonism-dementia complex of Guam, non-Guamanian motor neuron disease with neurofibrillary tangles, argyrophilic grain disease, corticobasal degeneration, diffuse neurofibrillary tangles with calcification, frontotemporal dementia with Parkinsonism linked to chromosome 17, Hallervorden-Spatz disease, multiple system atrophy, Niemann-Pick disease type C, pallido-ponto-nigral degeneration, Pick's disease, progressive subcortical gliosis, progressive supranuclear palsy (PSP), subacute sclerosing panencephalitis, tangle only dementia, postencephalitic Parkinsonism, myotonic dystrophy, Tau panencephalopathy, AD-like with astrocytes, certain prion diseases (GSS with Tau), mutations in LRRK2, chronic traumatic encephalopathy, familial British dementia, familial Danish dementia, frontotemporal lobar degeneration, Guadeloupean Parkinsonism, neurodegeneration with brain iron accumulation, SLC9A6-related mental retardation, white matter tauopathy with globular glial inclusions, traumatic stress syndrome, epilepsy, Lewy body dementia (LBD), hereditary cerebral hemorrhage with amyloidosis (Dutch type), mild cognitive impairment (MCI), multiple sclerosis, Parkinson's disease, atypical parkinsonism, HIV-related dementia, adult onset diabetes, senile cardiac amyloidosis, endocrine tumors, glaucoma, ocular amyloidosis, primary retinal degeneration, macular degeneration (such as age-related macular degeneration (AMD)), optic nerve drusen, optic neuropathy, optic neuritis, and lattice dystrophy. Preferably the diseases and conditions which can be detected and monitored include Alzheimer's disease (AD), familial AD, Creutzfeldt-Jacob disease, dementia pugilistica, Down's Syndrome, Gerstmann-Straussler-Scheinker disease, inclusion-body myositis, prion protein cerebral amyloid angiopathy, traumatic brain injury (TBI), amyotrophic lateral sclerosis, Parkinsonism-dementia complex of Guam, non-Guamanian motor neuron disease with neurofibrillary tangles, argyrophilic grain disease, corticobasal degeneration (CBD), diffuse neurofibrillary tangles with calcification, frontotemporal dementia with Parkinsonism linked to chromosome 17, Hallervorden-Spatz disease, multiple system atrophy, Niemann-Pick disease type C, pallido-ponto-nigral degeneration, Pick's disease (PiD), progressive subcortical gliosis, progressive supranuclear palsy (PSP), subacute sclerosing panencephalitis, tangle only dementia, postencephalitic Parkinsonism, myotonic dystrophy, Tau panencephalopathy, AD-like with astrocytes, certain prion diseases (GSS with Tau), mutations in LRRK2, chronic traumatic encephalopathy, familial British dementia, familial Danish dementia, frontotemporal lobar degeneration, Guadeloupean Parkinsonism, neurodegeneration with brain iron accumulation, SLC9A6-related mental retardation, and white matter tauopathy with globular glial inclusions, more preferably Alzheimer's disease (AD), Creutzfeldt-Jacob disease, dementia pugilistica, amyotrophic lateral sclerosis, argyrophilic grain disease, corticobasal degeneration, frontotemporal dementia with Parkinsonism linked to chromosome 17, Pick's disease, progressive supranuclear palsy (PSP), tangle only dementia, Parkinson dementia complex of Guam, Hallervorden-Spatz disease and fronto-temporal lobar degeneration. Preferably the disease or condition is Alzheimer's disease, Parkinson's disease or atypical parkinsonism, progressive supranuclear palsy (PSP), or Pick's disease (PiD), more preferably Alzheimer's disease.

Further examples of diseases or conditions that can be detected and monitored with the detectably labeled compounds obtained by the method of the present invention include Huntington's disease, ischemic stroke and psychosis in AD.

The method of the present invention has a number of important advantages compared to the prior art methods. Since only a single chromatographic step without subsequent reformulation via SPE is required after the compound of the formula I has been prepared the setup is much simpler than the prior art setups in which two different chromatographic steps are conducted.

The method has proven to be very robust. Due to the choice of ethanol and water as the mobile phase with an adjusted pH, larger quantities (e.g. > 1 mg) of precursor can be used without precipitation being caused.

The ¹⁸F-labelled compound of the formula I can be reliably separated from the precursor compound of the formula II and possible side products.

Furthermore, high yields and purities can be achieved. For instance, it is possible to obtain non-decay corrected yields of at least about 35%. The product activity can be at least about 20 GBq, preferably at least about 50 GBq, more preferably at least about 100 GBq. Radiochemical purities can be at least about 95%, preferably at least about 98% at low as well as at high radioactivity levels (at, e.g., at least about 100 GBq).

The present invention will be illustrated by the following examples which should not be construed as limiting.

### Examples

### Abbreviations

| | |
|---|---|
| AD | Alzheimer's disease |
| Boc, BOC | *tert*-butyloxycarbonyl |
| CBD | corticobasal degeneration |
| d.c. | corrected for decay |
| d | doublet |
| dd | doublet of doublet |
| DMF | *N,N*-dimethyl formamide |
| DMSO | dimethylsulfoxide |
| DMTrt-Cl | 4,4'-(chloro(phenyl)methylene)bis(methoxybenzene) |
| dppf | 1,1'-bis(diphenylphosphino)ferrocene |
| El | electron ionisation |
| ELSD | evaporative light scattering detector |
| EOS | End of synthesis |
| ESI | electrospray ionisation |
| EtOH | ethanol |
| FTD | Frontotemporal dementia |
| HPLC | high performance liquid chromatography |
| HC | Healthy control |
| GBq | Gigabequerel |
| K₂₂₂ | 4, 7, 13, 16, 21, 24-hexaoxa-1,10-diazabicyclo[8.8.8]-hexacosane (Kryptofix 222) |
| MBq | Megabequerel |
| MS | mass spectrometry |
| MeCN | acetonitrile |
| m | multiplet |
| mc | centered multiplet |
| n.c.a. | non-carrier -added |
| n.d.c. | not decay corrected |
| NMR | nuclear magnetic resonance spectroscopy : chemical shifts (δ) are given in ppm. |
| PET | Positron-Emission-Tomography |
| PiD | Pick's disease |
| PSP | progressive supranuclear palsy |
| q | quadruplet (quartet) |
| RT | room temperature |
| s | singulet |
| t | triplet |
| Tau | Tau protein, Tau deposits, Tau aggregates |
| TBI | Traumatic brain injury |
| TLC | thin layer chromatography |

### Experimental data

All reagents and solvents were obtained from commercial sources and used without further purification. Proton (¹H) spectra were recorded on a Bruker DRX-400 MHz NMR spectrometer or on a Bruker AV-400 MHz NMR spectrometer in deuterated solvents. Mass spectra (MS) were recorded on an Advion CMS mass spectrometer. Chromatography was performed using silica gel (Fluka: Silica gel 60, 0.063-0.2 mm) and suitable solvents as indicated in the specific examples. Flash purification was conducted with a Biotage Isolera One flash purification system using HP-Sil (Biotage) or puriFlash-columns (Interchim) and the solvent gradient indicated in the specific examples. Thin layer chromatography (TLC) was carried out on silica gel plates with UV detection.

### SYNTHESIS OF TEST COMPOUNDS

### Preparative Example A

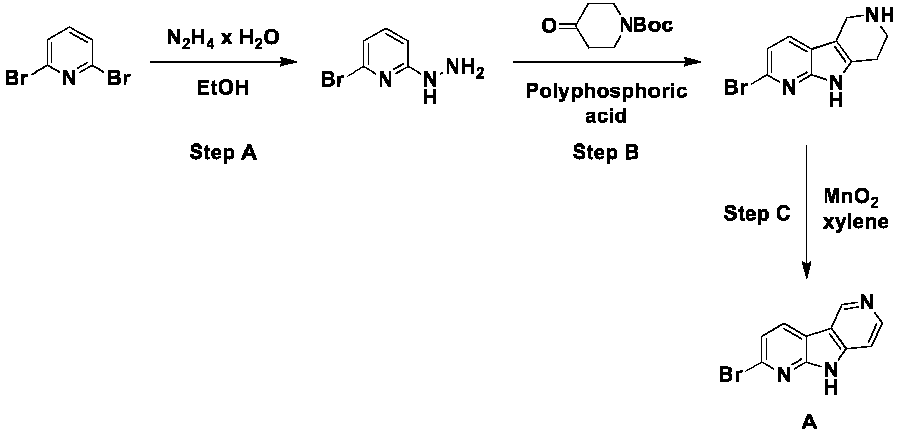

### Step A

Commercially available 2,6-dibromopyridine (4.12 g, 16.6 mmol) was suspended in ethanol (40 mL) and hydrazine hydrate (10 mL, 97.6 mmol) in water (∼50-60 %) was added. The mixture was heated in a sand-bath at ∼115 °C for 18 hours. The solvent was removed and the residue was purified by chromatography on silica using ethyl acetate/n-heptane (60/40) to afford the title compound as an off-white solid (3.05 g, 93 %).

¹H-NMR (400 MHz, CDCl₃): δ = 7.33 (t, 1H), 6.83 (d, 1H), 6.67 (d, 1H), 6.00 (br-s, 1H), 3.33-3.00 (br-s, 2H)

### Step B

The title compound from Step A above (10 g, 53.2 mmol) and commercially available 1-Boc-4-piperidone (10.6 g, 53.2 mmol) were added to a 500 mL flask and mixed to become a homogenous blend. Then polyphosphoric acid (80 g, 115% H₃PO₄ basis) was added and the mixture was heated at ∼160°C in a sand-bath. At ∼120°C the Boc-protecting group was cleaved resulting in foaming of the reaction mixture. After complete Boc-cleavage the foam collapsed and the dark reaction mixture was stirred at ∼160°C for 20 hours. The reaction mixture was allowed to cool to room temperature and water (400 mL) was added. The reaction mixture was stirred/sonicated until the gummy material was dissolved. The reaction mixture was then placed in an ice-bath and the pH of the solution was adjusted to pH -12 by adding solid sodium hydroxide pellets (exothermic). The precipitate was collected by filtration and washed with water (400 mL) to remove salts. The precipitate was dissolved in dichloromethane/methanol (9/1; 1500 mL) by sonication and washed with water (2 x 400 mL) to remove remaining salts and insoluble material. The organic phase was dried over Na₂SO₄, filtered and the solvents were removed under reduced pressure. The dark residue was treated with dichloromethane (100 mL), sonicated for 5 minutes and the precipitate was collected by filtration. The precipitate was washed with dichloromethane (40 mL) and air-dried to afford the title compound as a beige solid (3.5 g, 26 %).

¹H-NMR (400 MHz, DMSO-d₆): δ = 11.5 (br-s, 1H), 7.72 (d, 1H), 7.15 (d, 1H), 3.86-3.82 (m, 2H), 3.06-3.00 (m, 2H), 2.71-2.65 (m, 2H)

### Step C

The title compound from Step B above (1.75 g, 6.94 mmol) was suspended in xylene (380 mL) and manganese (IV) oxide (6.62 g, 76.9 mmol) was added. The reaction mixture was then heated at ∼160°C in a sand-bath for 36 hours. The cooled reaction mixture was evaporated under reduced pressure, the residue was suspended in dichloromethane/methanol (1/1; 400 mL) and stirred at room temperature for 30 minutes. The reaction mixture was then filtered through paper filters to remove the manganese (IV) oxide and the filter was washed with methanol (50 mL). The combined filtrates were evaporated under reduced pressure and the dark residue was purified by chromatography on silica (50 g HP-SIL-cartridge) using a Biotage Isolera system employing an ethyl acetate/heptane gradient (5/95-100/0) to remove unpolar impurities followed by dichloromethane/methanol (9/1 -> 4/1) to afford the title compound as a dark yellow solid. The total yield from 2 runs was 1.77 g (51 %).

¹H-NMR (400 MHz, DMSO-d₆): δ = 12.52 (br-s, 1H), 9.42 (s, 1H), 8.61 (d, 1H), 8.53 (d, 1H), 7.56-7.52 (m, 2H)

### Preparative Example B

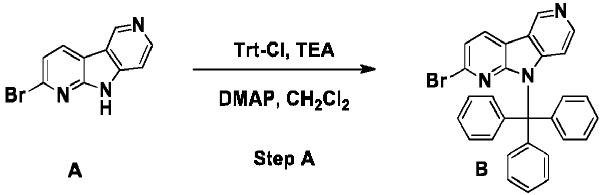

### Step A

To a suspension of the title compound from Preparative Example **A** (0.776 g, 0.72 mmol) in dichloromethane (65 mL) was added triethylamine (1.86 mL, 13 mmol) and trityl-chloride (2.63 g, 9.39 mmol). After the addition of 4-(dimethylamino)-pyridine (0.074 g, 0.608 mmol), the reaction mixture was stirred at room temperature for 16 hours. The reaction mixture was diluted with dichloromethane (150 mL) and water (50 mL). The organic phase was separated, dried over Na₂SO₄, filtered and the solvents were removed in *vacuo.* The residue was purified on HP-Sil SNAP cartridges (50 g) using a Biotage Isolera One purification system employing an ethyl acetate/n-heptane gradient (5/95 -> 100/0 -> 100/0) to afford the title compound B as a pale yellow solid (0.831 g, 54 %). Unreacted starting material was recovered by flushing the cartridge with ethyl acetate/methanol (90/10) to afford the starting material as an off-white solid (0.195 g, 25 %).

¹H-NMR (400 MHz, CDCl₃) δ = 9.22 (s, 1H), 8.23 (d, 1H), 8.13 (d, 1H), 7.48-7.42 (m, 7H), 7.33-7.22 (m, 12H), 6.41 (d, 1H)

MS (ESI); m/z = 490.03/491.96 [M+H]⁺

### Preparative Example C

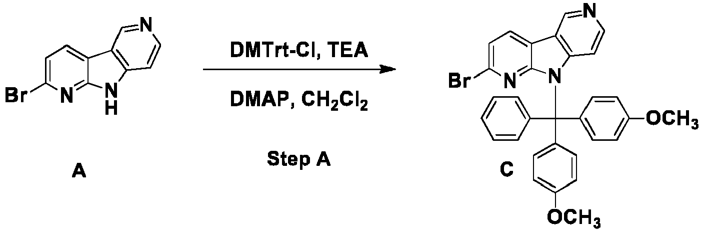

### Step A

To a suspension of the title compound from Preparative Example A (0.482 g, 1.94 mmol) in dichloromethane (40 mL) was added triethylamine (1.15 mL, 8 mmol) and 4,4'-(chloro(phenyl)methylene)bis(methoxybenzene) (DMTrt-Cl) (1.963 g, 5.8 mmol). After the addition of 4-(dimethylamino)-pyridine (0.046 g, 0.377 mmol), the reaction mixture was stirred at room temperature for 3 days. The reaction mixture was diluted with dichloromethane (100 mL) and water (40 mL). The organic phase was separated, dried over Na₂SO₄, filtered and the solvents were removed in *vacuo.* The residue was purified on HP-Sil SNAP cartridges (50 g) using a Biotage Isolera One purification system employing an ethyl acetate/n-heptane gradient (5/95 -> 100/0 -> 100/0) to afford the title compound C as a pale yellow solid (0.825 g, 72 %). Unreacted starting material was recovered by flushing the cartridge with ethyl acetate/methanol (90/10) to afford the starting material as an off-white solid (0.042 g, 8.8 %).

¹H-NMR (400 MHz, CDCl₃) δ = 9.23 (s, 1H), 8.23 (d, 1H), 8.13 (d, 1H), 7.39-7.31 (m, 6H), 7.29-7.25 (4H), 6.80 (d, 4H), 6.41 (dd, 1H), 3.81 (s, 6H)

### Example 1

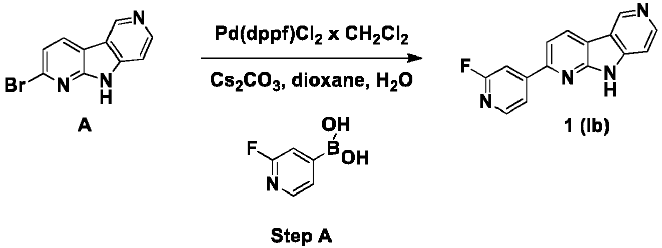

### Step A

To a mixture of degassed 1,4-dioxane (4.3 mL) and water (1 mL) in a microwave vial was added [1,1'-bis(diphenylphosphino)ferrocene]dichloropalladium(II), complex with dichloromethane (0.0084 g, 0.01 mmol), followed by the title compound of Preparative Example A (0.05 g, 0.2 mmol), (2-fluoropyridin-4-yl)boronic acid (0.035 g, 0.245 mmol) and cesium carbonate (0.133 g, 0.41 mmol). The reaction mixture was then heated at ∼115°C in a sand-bath for 6 hours. The reaction mixture was diluted with ethyl acetate (60 mL) and water (20 mL), the organic phase was separated, dried over Na₂SO₄, filtered and the solvents were evaporated *in vacuo.* The dark residue was purified by chromatography on silica (25 g HP-SIL) using a Biotage Isolera system employing a dichloromethane/methanol gradient (100/0 -> 95/5 -> 90/10 -> 80/20) to afford the title compound 1 as an off-white solid (0.033 g, 63 %).

¹H-NMR (400 MHz, DMSO-d₆) δ = 12.50 (br-s, 1H), 9.45 (s, 1H), 8.83 (d, 1H), 8.56-8.52 (m, 1H), 8.43-8.39 (m, 1H), 8.19-8.14 (m, 2H), 7.92 (s, 1H), 7.54-7.50 (m, 1H)

MS (ESI): m/z = 265.04 [M+H]⁺

### Example 2

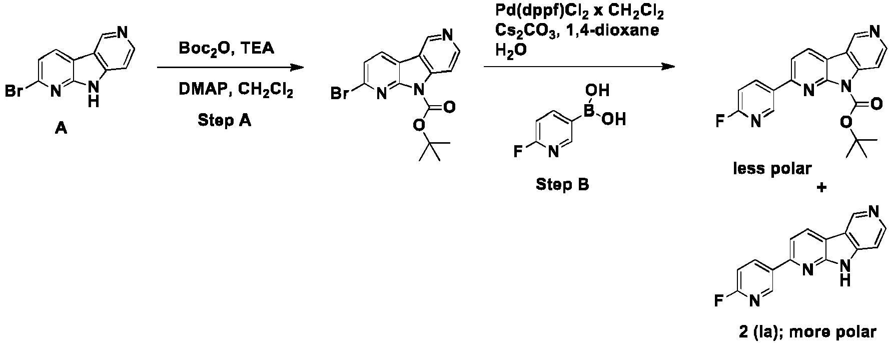

### Step A

To a suspension of the title compound of Preparative Example A (0.430 g, 1.73 mmol) in dichloromethane (25 mL) was added triethylamine (1.93 mL, 13.89 mmol) and di-tert-butyl dicarbonate (2.27 g, 10.02 mmol). After the addition of 4-(dimethylamino)-pyridine (0.042 g, 0.34 mmol), the reaction mixture was stirred at room temperature for 3 days. The solvents were removed under reduced pressure and the residue was purified on HP-Sil SNAP cartridges (25 g) using a Biotage Isolera One purification system employing an ethyl acetate/n-heptane gradient (5/95 -> 100/0 -> 100/0) to afford the title compound as off-white solid (0.558 g, 92 %).

¹H-NMR (400 MHz, CDCl₃) δ = 9.28 (s, 1H), 8.73 (d, 1H), 8.22 (d, 2H), 7.59 8d, 1H), 1.80 (s, 9H)

### Step B

To a mixture of degassed 1,4-dioxane (3 mL) and water (0.7 mL) in a microwave vial was added [1,1'-bis(diphenylphosphino)ferrocene]dichloropalladium(II), complex with dichloromethane (0.0058 g, 0.007 mmol), followed by the title compound from Step A above (0.05 g, 0.143 mmol), (6-fluoropyridin-3-yl)boronic acid (0.024 g, 0.17 mmol) and cesium carbonate (0.092 g, 0.286 mmol). The reaction mixture was then heated at ∼100°C in a sand-bath for 4 hours. The reaction mixture was diluted with ethyl acetate (80 mL) and water (35 mL), the organic phase separated, dried over Na₂SO₄, filtered and the solvents were evaporated *in vacuo.* The dark residue was purified by chromatography on silica (12 g, puriFlash, Interchim) using a Biotage Isolera system employing a dichloromethane/methanol gradient (100/0 -> 98/2 -> 95/5 -> 90/10 -> 80/20) to afford the less polar Boc-protected compound (0.0255 g, 49 %) and the more polar title compound **2** as off-white solid (0.0116 g, 31 %). More polar title compound **2:**
¹H-NMR (400 MHz, DMSO-*d*₆) δ = 12.40 (br-s, 1H), 9.40 (s, 1H), 9.05 (s, 1H), 8.78-8.70 (m, 2H), 8.51 (d, 1H), 8.02 (d, 1H), 7.50 (d, 1H), 7.36 (dd, 1H)
MS (ESI): m/z = 265.09 [M+H]⁺

Less polar Boc-protected compound:
¹H-NMR (400 MHz, DMSO-*d*₆) δ = 9.48 (s, 1H), 9.13 (d, 1H), 8.84-8.78 (m, 2H), 8.68 (d, 1H), 8.23 (d, 1H), 8.19 (d, 1H), 7.40 (dd, 1H), 1.75 8s, 9H)

The synthesis of title compound **2** was first described in WO 2015/052105 (Example 1) by a different synthesis.

### SYNTHESIS OF RADIOLABELING PRECURSORS

### Example 3-a

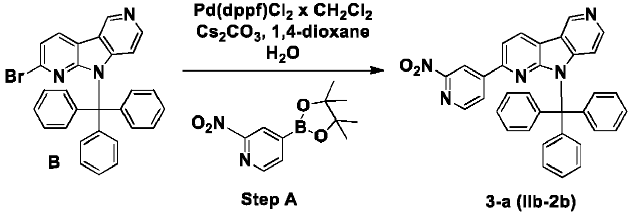

### Step A

To a mixture of degassed 1,4-dioxane (4.3 mL) and water (1 mL) in a microwave vial was added [1,1'-bis(diphenylphosphino)ferrocene]dichloropalladium(II), complex with dichloromethane (0.0084 g, 0.01 mmol), the title compound of Preparative Example B (0.1 g, 0.2 mmol), 2-nitro-4-(4,4,5,5-tetramethyl-1,3,2-dioxaborolan-2-yl)pyridine (0.061 g, 0.245 mmol) and cesium carbonate (0.133 g, 0.41 mmol). The reaction mixture was then heated at ∼115°C in a sand-bath for 6 hours. The reaction mixture was diluted with ethyl acetate (60 mL) and water (20 mL), the organic phase was separated, dried over Na₂SO₄, filtered and the solvents were evaporated *in vacuo.* The dark residue was purified by chromatography on silica (25 g pufiFlash-column, Interchim) using a Biotage Isolera system employing an ethyl acetate/n-heptane gradient (5/95 -> 100/0 -> 100/0) to afford the title compound 3-a as a pale-yellow solid (0.082 g, 75 %).

¹H NMR (400 MHz, CDCl₃) δ = 9.32 (s, 1H); 8.56 (d, 1H), 8.48 (d, 1H), 8.33 (s, 1H); 8.30 (d, 1H), 7.85 (d, 1H), 7.69 (d, 1H), 7.58-7.54 (m, 5H), 7.32-7.25 (m, 10H), 6.48 (d, 1H)

MS (ESI): m/z = 534.28 [M+H]⁺.

### Example 3-b

### Method a:

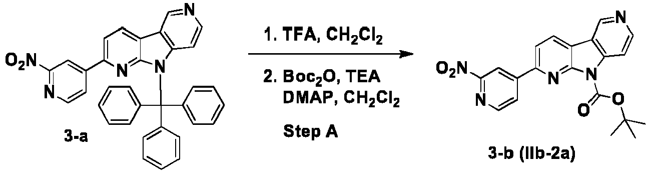

### Step A

To a solution of **3-a** (0.0396 g, 0.074 mmol) in dichloromethane (5 mL) was added trifluoroacetic acid (1.2 mL). The reaction mixture was stirred at room temperature for 6 hours and methanol (2 mL) was added. The solvents were evaporated *in vacuo* and the residue was dissolved/suspended in methanol (5 mL). The solvents were evaporated *in vacuo* and the residue again dissolved/suspended in methanol (5 mL). The solvents were evaporated *in vacuo* and the residue suspended in dichloromethane (2 mL). After the addition of triethylamine (1 mL, 7.2 mmol), di-*tert*-butyl dicarbonate (0.098 g, 0.43 mmol), and 4-(dimethylamino)-pyridine (0.0018 g, 0.014 mmol), the reaction mixture was stirred at room temperature for 18 hours. The reaction mixture was diluted with ethyl acetate (50 mL) and water (20 mL). The organic phase was separated, dried over Na₂SO₄, filtered and the solvents removed in *vacuo.* The residue was purified on silica (25 g puriFlash, Interchim) using a Biotage Isolera One purification system employing an ethyl acetate/n-heptane gradient (5/95 -> 100/0 -> 100/0) to elute unpolar by-products followed by ethyl acetate/methanol (95/5) to afford the title compound **3-b** pale as a yellow solid (0.0184 g, 63 %).

¹H-NMR (400 MHz, CDCl₃) δ = 9.36 (s, 1H), 9.15 (s, 1H), 8.82-8.76 (m, 2H), 8.57 (d, 1H), 8.45 (d, 1H), 8.36 (d, 1H), 8.07 (d, 1H), 1.87 (s, 9H)

MS (ESI); m/z = 391.82 [M+H]⁺

### Method b:

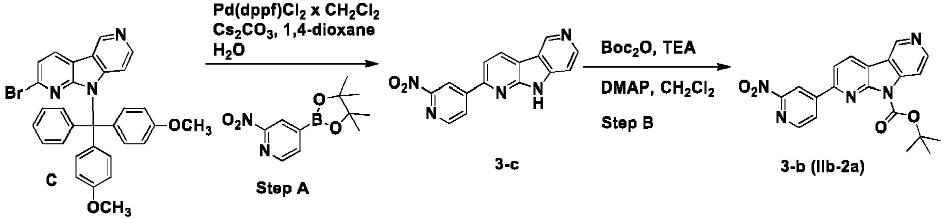

### Step A

To a mixture of degassed 1,4-dioxane (2.2 mL) and water (0.5 mL) in a microwave vial was added [1,1'-bis(diphenylphosphino)ferrocene]dichloropalladium(II), complex with dichloromethane (0.0042 g, 0.005 mmol), followed by the title compound of Preparative Example C (0.055 g, 0.1 mmol), 2-nitro-4-(4,4,5,5-tetramethyl-1,3,2-dioxaborolan-2-yl)pyridine (0.0305 g, 0.12255 mmol) and cesium carbonate (0.067 g, 0.205 mmol). The reaction mixture was then heated at ∼115°C in a sand-bath for 6 hours. The reaction mixture was diluted with ethyl acetate (20 mL), the precipitate collected by filtration, washed with water (10 mL) and methanol (5 mL) and air dried to afford **3-c** (0.0277 g, 95%).

### Step B

To a suspension of the crude title compound from Step A above (0.0277 g, 0.095 mmol) in dichloromethane (4 mL) were added triethylamine (1 mL, 7.2 mmol), di-tert-butyl dicarbonate (0.2 g, 0.86 mmol), and 4-(dimethylamino)-pyridine (0.0036 g, 0.028 mmol). The reaction mixture was stirred at room temperature for 16 hours, diluted with ethyl acetate (50 mL) and water (20 mL). The organic phase was separated, dried over Na₂SO₄, filtered and the solvents were removed in *vacuo.* The residue was purified on silica (25 g puriFlash, Interchim) using a Biotage Isolera One purification system employing an ethyl acetate/n-heptane gradient (5/95 -> 100/0 -> 100/0) to elute unpolar by-products followed by ethyl acetate/methanol (95/5) to afford the title compound 3-b as a pale yellow solid (0.0261 g, 70 %).

¹H-NMR (400 MHz, CDCl₃) δ = 9.38 (s, 1H), 9.16 (s, 1H), 8.83-8.78 (m, 2H), 8.58 (d, 1H), 8.46 (d, 1H), 8.38 (d, 1H), 8.09 (d, 1H), 1.88 (s, 9H)

MS (ESI); m/z = 391.85 [M+H]⁺; 291.74 [M+H-Boc]⁺

### Example 3-d

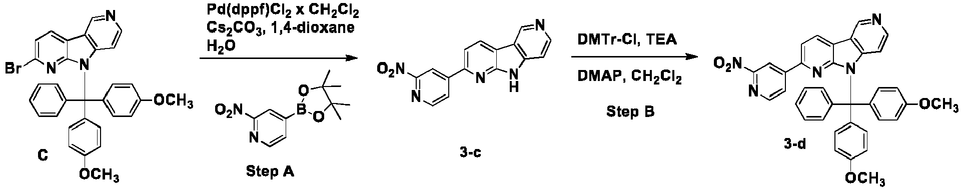

### Step A

To a mixture of degassed 1,4-dioxane (2.2 mL) and water (0.5 mL) in a microwave vial was added [1,1'-bis(diphenylphosphino)ferrocene]dichloropalladium(II), complex with dichloromethane (0.0042 g, 0.005 mmol), followed by the title compound of Preparative Example C (0.055 g, 0.1 mmol), 2-nitro-4-(4,4,5,5-tetramethyl-1,3,2-dioxaborolan-2-yl)pyridine (0.0305 g, 0.12255 mmol) and cesium carbonate (0.067 g, 0.205 mmol). The reaction mixture was then heated at ∼115°C in a sand-bath for 6 hours. The reaction mixture was diluted with ethyl acetate (20 mL), the precipitate collected by filtration, washed with water (10 mL) and methanol (5 mL) and air dried to afford 3-c as a grey solid (0.0277 g, 95%).

### Step B

To a suspension of the crude title compound from Step A above (0.0277 g, 0.095 mmol) in dichloromethane (4 mL) was added triethylamine (1 mL, 7.2 mmol), 4,4'-(chloro(phenyl)methylene)bis(methoxybenzene) (0.081 g, 0.29 mmol), and 4-(dimethylamino)-pyridine (0.0036 g, 0.028 mmol). The reaction mixture was stirred at room temperature for 18 hours, diluted with ethyl acetate (50 mL) and water (20 mL). The organic phase was separated, dried over Na₂SO₄, filtered and the solvents were removed in *vacuo.* The residue was purified on silica (25 g puriFlash, Interchim) using a Biotage Isolera One purification system employing an ethyl acetate/n-heptane gradient (5/95 -> 100/0 -> 100/0) to afford the title compound 3-d as a pale yellow solid (0.0261 g, 44 %).

¹H-NMR (400 MHz, CDCl₃) δ = 9.32 (s, 1H), 8.58 (d, 1H), 8.50 (d, 1h), 8.36 (s, 1H), 8.30 (d, 1H), 7.85 (d, 1H), 7.74 (d, 1H), 7.52-7.42 (m, 6H), 7.27-7.23 (m, 4H), 6.80 (d, 4H), 6.49 (d, 1H), 3.78 (s, 6H)

### Example 3-e

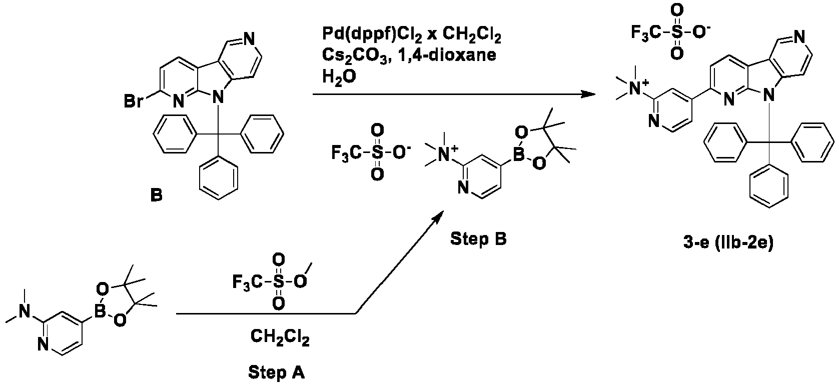

### Step A

Commercially available N,N-dimethyl-4-(4,4,5,5-tetramethyl-1,3,2-dioxaborolan-2-yl)pyridin-2-amine (0.25 g, 1 mmol) was dissolved in dichloromethane (5 mL). To the resultant stirring solution was added dropwise at room temperature methyl trifluoromethanesulfonate (0.124 mL, 1.1 mmol). The solution was stirred at room temperature for 4 hours. The reaction mixture was concentrated to remove dichloromethane and the residue was dried in vacuo to obtain a yellow glass/foam, which was directly used for the next step.

### Step B

To a solution of degassed 1,4-dioxane (12 mL) and water (3 mL) in a microwave vial was added [1,1'-bis(diphenylphosphino)ferrocene]dichloro-palladium(II), complex with dichloromethane (0.034 g, 0.04 mmol), the title compound of Preparative Example B (0.4 g, 0.816 mmol), the crude title compound from Step A above (∼1 mmol) and cesium carbonate (0.544 g, 1.68 mmol). The reaction mixture was heated at ∼120°C in a sand-bath for 6 hours. The reaction mixture was diluted with ethyl acetate (150 mL) and water (50 mL), the organic phase separated, dried over Na₂SO₄, filtered and the solvents were evaporated in vacuo. The dark residue was purified by chromatography on silica (25 g HP-Ultra) using a Biotage Isolera system employing an ethyl acetate/n-heptane gradient (5/95 -> 100/0 -> 100/0) to elute unreacted starting material and unpolar by-products. The gradient was then changed to dichloromethane/methanol (100/0 -> 95/5 -> 90/10) to afford the dimethylamine-derivative as a pale yellow glass (0.127 g, 29%; MS (ESI): m/z = 532.27 [M+H]+) and the methylamine-derivative as a grey solid (0.0547 g, 13%; MS (ESI): m/z = 519.18 [M+H]+). The gradient was again changed to dichloromethane/methanol (90/10 -> 80/20) and held at (80/20) to obtain the title compound 3-e as a brown solid (0.104 g, 18%).

¹H NMR (400 MHz, DMSO-d6) δ = 9.47 (s, 1H); 8.89 (d, 1H), 8.55 (d, 1H), 8-35-8.32 (m, 2H), 8.29 (d, 1H), 7.63-7.57 (m, 5H), 7.48 (d, 1H), 7.34-7.25 (m, 10H), 6.48 (d, 1H), 3.60 (s, 9H) MS (ESI): m/z = 546.26 [M+H]⁺

### Example 3-f

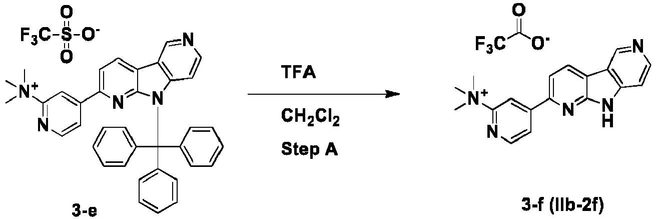

### Step A

**3-e** (0.199 g, 0.364 mmol) was suspended in dichloromethane (10 mL). After the addition of trifluoro acetic acid (10 mL), the reaction mixture was stirred at room temperature for 18 hours. The solvents were removed under reduced pressure, the residue dissolved in methanol (10 mL) and the solvents were removed under reduced pressure. The methanol treatment of the residue was repeated two more times. The residue was then suspended in dichloromethane (20 mL) and sonicated for ∼5 minutes. The precipitate was collected by filtration, washed with dichloromethane (10 mL) and air-dried to afford the title compound 3-f as a grey solid (0.127 g, 83%).

¹H NMR (400 MHz, DMSO-d6) δ = 13.76 (br-s, 1H), 9.84 (s, 1H); 8.12 (d, 1H), 8.89 (d, 1H), 8.80 (d, 1H), 8.75 (s, 1H), 8.54-8.50 (m, 2H), 8.04 (d, 1H), 3.72 (s, 9H)

MS (ESI): m/z = 303.91 [M+H]⁺

### Example 4-a

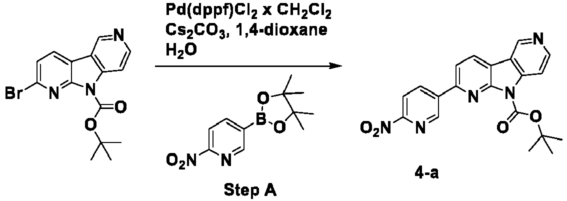

### Step A

To a mixture of degassed 1,4-dioxane (3 mL) and water (0.7 mL) in a microwave vial was added [1,1'-bis(diphenylphosphino)ferrocene]dichloropalladium(II), complex with dichloromethane (0.0058 g, 0.007 mmol), followed by the title compound from Example 2 Step A (0.05 g, 0.143 mmol), 2-nitro-5-(4,4,5,5-tetramethyl-1,3,2-dioxaborolan-2-yl)pyridine (0.0428 g, 0.17 mmol) and cesium carbonate (0.092 g, 0.286 mmol). The reaction mixture was then heated at ∼100°C in a sand-bath for 4 hours. The reaction mixture was diluted with ethyl acetate (80 mL) and water (35 mL), the organic phase separated, dried over Na₂SO₄, filtered and the solvents were evaporated *in vacuo.* The dark residue was purified by chromatography on silica (12 g, puriFlash, Interchim) using a Biotage Isolera system employing a dichloromethane/methanol gradient (100/0 -> 98/2 -> 95/5 -> 90/10 -> 80/20) to afford the title compound **4-a** as a pale yellow solid (0.0173 g, 31%).

¹H NMR (400 MHz, CDCl₃/CD₃OD) δ = 9.45 (d, 1H), 9.32 (s, 1H), 8.93 (dd, 1H), 8.68-8.64 (m, 2H), 8.46 (d, 1H), 8.35 (d, 1H), 8.14 (d, 1H), 1.82 (s, 9H)

MS (ESI): m/z = 392.13 [M+H]⁺

The synthesis of title compound **4-a** was first described in WO 2015/052105 (Example 3a) by a different synthesis.

### RADIOLABELING OF PRECURSORS WITH ¹⁸F

Examples of radiolabeling precursors

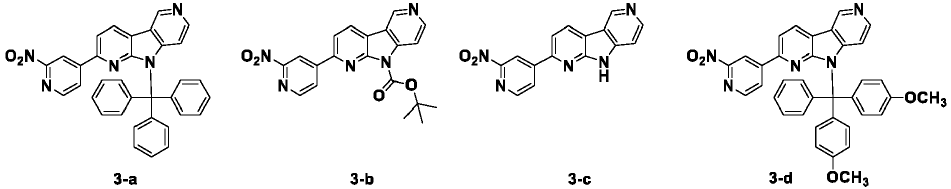

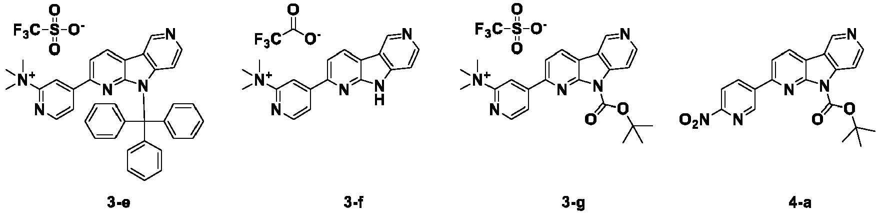

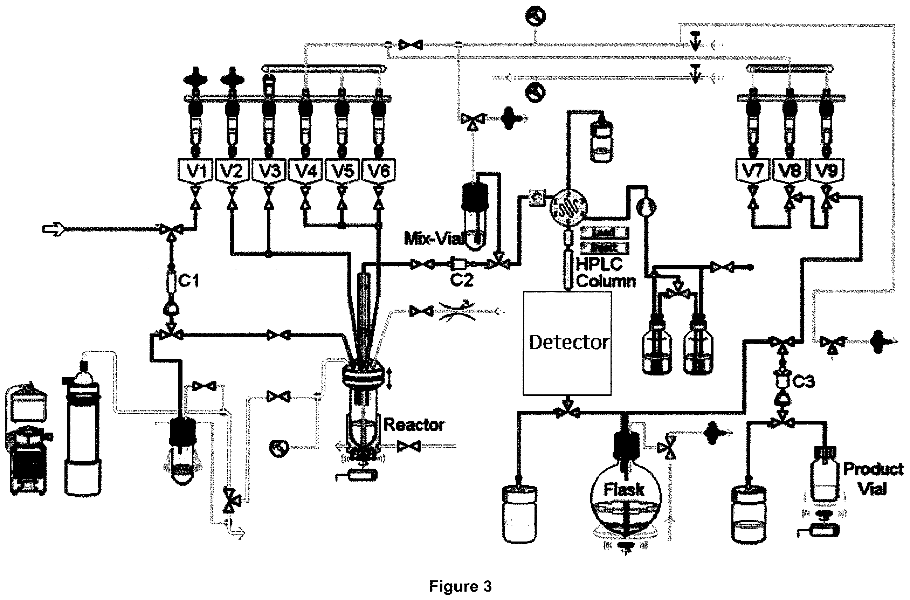

### General radiolabeling method A, performed on a tracerlab FX such as illustrated in Figure 3 (Radiolabeling, deprotection, HPLC and SPE) - Comparative Examples

[¹⁸F]fluoride was trapped on a Sep-Pak Accell Plus QMA light cartridge (Waters) and eluted with a solution K₂CO₃/Kryptofix^{®} 2.2.2. The water was removed using a stream of He or N₂ at 95 °C and co-evaporated to dryness with MeCN (1 mL). Afterwards, a solution of the dissolved precursor was added to the dried K[¹⁸F]F-Kryptofix complex. The reaction vial was sealed and heated for 15 min at 150 °C. Subsequently, an acid (HCI, H₂SO₄ or H₃PO₄) was added and the mixture was heated for 10 min at 150 °C. The reaction mixture was diluted with 1 mL NaOH and 2.4 mL of the prep. HPLC mobile phase and the crude product was purified via semi-preparative HPLC (e.g. Phenomenex, Gemini C18, 5 µm, 250 x 10 mm) at 4 mL/min. The isolated tracer was diluted with water (20 mL + 10 mg/mL sodium ascorbate), trapped on a C-18 Plus cartridge (Waters), washed with water (10 mL + 10 mg/mL sodium ascorbate), eluted with ethanol (1 mL) and mixed with water (14 mL+10 mg/mL sodium ascorbate).

### General radiolabeling method B, performed on a tracerlab FX such as illustrated in Figure 3 (Radiolabeling, HPLC and SPE) - Comparative Examples

[¹⁸F]fluoride was trapped on a Sep-Pak Accell Plus QMA light cartridge (Waters) and eluted with a solution K₂CO₃/Kryptofix^{®} 2.2.2. The water was removed using a stream of He or N₂ at 95 °C and co-evaporated to dryness with MeCN (1 mL). Afterwards, a solution of the dissolved precursor was added to the dried K[¹⁸F]F-Kryptofix complex. The reaction vial was sealed and heated for 15 min at 150 °C. The reaction mixture was diluted with 0.5-1 mL NaOH and 2.4 mL of the prep. HPLC mobile phase and the crude product was purified via semi-preparative HPLC (e.g. Phenomenex, Gemini C18, 5 µm, 250 x 10 mm) at 4 mL/min. The isolated tracer was diluted with water (20 mL + 10 mg/mL sodium ascorbate), trapped on a C-18 Plus cartridge (Waters), washed with water (10 mL + 10 mg/mL sodium ascorbate), eluted with ethanol (1 mL) and mixed with water (14 mL+10 mg/mL sodium ascorbate).

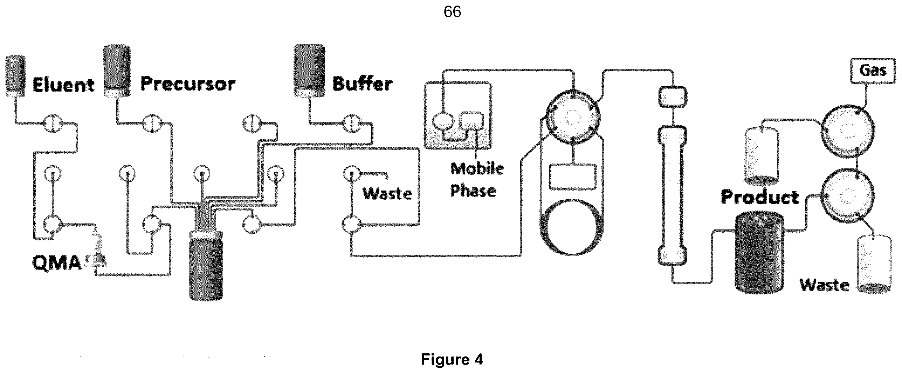

### General radiolabeling method C performed on a IBA Synthera + Synthera HPLC such as illustrated in Figure 4 (Radiolabeling, HPLC)

[¹⁸F]fluoride was trapped on a Sep-Pak Accell Plus QMA light cartridge (Waters) and eluted with a solution K₂CO₃/Kryptofix^{®} 2.2.2. The water was removed using a stream of He or N₂ at 95-110 °C and co-evaporated to dryness. Afterwards, a solution of the dissolved precursor was added to the dried K[¹⁸F]F-Kryptofix complex. The reaction vial was sealed and heated for 15 min at 150 °C. The reaction mixture was diluted with 0.5-1 mL 1M H₃PO₄ and 3-3.5 mL of the aqueous component of the prep. HPLC mobile phase and the crude product was purified via semi-preparative HPLC (e.g. Waters XBridge Peptide BEH C18, 130 Å, 10 µm, 10 mm x 250 mm) at 3-6 mL/min. The fraction containing the product (5-10 mL) was collected and diluted with a dilution media containing 0-2 mL EtOH, 10-20 mL water, and 0-4 mL phosphate buffer concentrate (Braun, 3.05 g of disodium monohydrogen phosphate dodecahydrate, 0.462 g of sodium dihydrogen phosphate dihydrate in 20 mL of water for injection) and/or sodium ascorbate (100-1000 mg) and/or sodium citrate (100-1000 mg) and/or gentisic acid (20-200 mg).

### General radiolabeling method D, performed on a tracerlab FX such as illustrated in Figure 3 (Radiolabeling, HPLC)

[¹⁸F]fluoride was trapped on a Sep-Pak Accell Plus QMA light cartridge (Waters) and eluted with a solution K₂CO₃/Kryptofix^{®} 2.2.2. The water was removed using a stream of He or N₂ at 95 °C and co-evaporated to dryness with MeCN (1 mL). Afterwards, a solution of the dissolved precursor was added to the dried K[¹⁸F]F-Kryptofix complex. The reaction vial was sealed and heated for 15 min at 150 °C. The reaction mixture was diluted with 0.5-1 mL 1M H₃PO₄ and 3-3.5 mL of the aqueous component of the prep. HPLC mobile phase and the crude product was purified via semi-preparative HPLC (e.g. Waters XBridge Peptide BEH C18, 130 Å, 10 µm, 10 mm x 250 mm or Gemini 5 µm C18, 250x10 mm, Phenomenex: 00G-4435-N0) at 3-6 mL/min. The fraction containing the product (5-10 mL) was collected and diluted with a dilution media containing 0-2 mL EtOH, 10-20 mL water, and 0-4 mL phosphate buffer concentrate (Braun) and/or sodium ascorbate (100-1000 mg) and/or sodium citrate (100-1000 mg) and/or gentisic acid (20-200 mg).

### General radiolabeling method E, performed on a tracerlab FX such as illustrated in Figure 3 (Radiolabeling, deprotection, HPLC)

[¹⁸F]fluoride was trapped on a Sep-Pak Accell Plus QMA light cartridge (Waters) and eluted with a solution K₂CO₃/Kryptofix^{®} 2.2.2. The water was removed using a stream of He or N₂ at 95 °C and co-evaporated to dryness with MeCN (1 mL). Afterwards, a solution of the dissolved precursor was added to the dried K[¹⁸F]F-Kryptofix complex. The reaction vial was sealed and heated for 15 min at 150 °C. Subsequently, 1 mL 0.5M H₂SO₄ was added and the mixture was heated for 10 min at 100 °C. The reaction mixture was diluted with 0.5-1 mL 1M NaOH and 2-3 mL of the aqueous component of the prep. HPLC mobile phase and the crude product was purified via semi-preparative HPLC (e.g. Waters XBridge Peptide BEH C18, 130 Å, 10 µm, 10 mm x 250 mm or Gemini 5 µm C18, 250x10 mm, Phenomenex: 00G-4435-N0) at 3-6 mL/min. The fraction containing the product (5-10 mL) was collected and diluted with a dilution media containing 0-2 mL EtOH, 10-20 mL water, and 0-4 mL phosphate buffer concentrate (Braun) and/or sodium ascorbate (100-1000 mg) and/or sodium citrate (100-1000 mg) and/or gentisic acid (20-200 mg).

### General radiolabeling method F, performed on an IBA Synthera + Synthera HPLC such as illustrated in Figure 4 (Radiolabeling, deprotection, HPLC)

[¹⁸F]fluoride was trapped on a Sep-Pak Accell Plus QMA light cartridge (Waters) and eluted with a solution K₂CO₃/Kryptofix^{®} 2.2.2. The water was removed using a stream of He or N₂ at 95-110 °C and co-evaporated to dryness. Afterwards, a solution of the dissolved precursor was added to the dried K[¹⁸F]F-Kryptofix complex. The reaction vial was sealed and heated for 5-10 min at 110-120 °C. After fluorination, 0.5-1 mL 1M H₃PO₄ was added and the reaction mixture heated for 10-15 min at 100-110 °C. The mixture was diluted with 3-3.5 mL of the aqueous component of the prep. HPLC mobile phase and the crude product was purified via semi-preparative HPLC (e.g. Waters XBridge Peptide BEH C18, 130 Å, 10 µm, 10 mm x 250 mm) at 3-6 mL/min. The fraction containing the product (5-10 mL) was collected and diluted with a dilution media containing 0-2 mL EtOH, 10-20 mL water, and 0-4 mL phosphate buffer concentrate (Braun, 3.05 g of disodium monohydrogen phosphate dodecahydrate, 0.462 g of sodium dihydrogen phosphate dihydrate in 20 mL of water for injection) and/or sodium ascorbate (100-1000 mg) and/or sodium citrate (100-1000 mg) and/or gentisic acid (20-200 mg).

### Determination of the radiochemical purity

Radiochemical purity was determined by analytical HPLC, e.g.: column: Atlantis T3, Waters, 100 x 4.6 mm, 3 µm, 100; mobile phase A: 40 mM sodium acetate, finally adjusted to pH 5.0 with glacial acetic acid; mobile phase B: 35% methanol in acetonitrile; flow rate: 1.8 mL/min; gradient: 0-5 min 15-32% B, 5-8 min 32-80% B, 8-12 min 80% B, 12-13 min 80-15% B, 13-16 min 15% B.

Results for synthesis of ¹⁸F-lb:

| **#** | **Precursor** | **General Radiolabeling method** | **prep. HPLC mobile phase** | **Overall synthesis time [min]** | **Start Activity [GBq]** | **Product Activity [GBq]** | **Non-decay corrected yield [%]** | **Radiochemical purity at EOS [%]** |
|---|---|---|---|---|---|---|---|---|
| 1* | 2 mg **3a** in 0.8 mL DMSO | A | 23% MeCN, 77% NaH₂PO₄/ Na₂HPO₄ buffer | ≈ 74 | 375 | 36 | 9.6 | 97.6 |
| 2 | 2 mg **3b** in 1 mL DMSO | C | 15% EtOH, 85% 100 mM NaH₂PO₄ (adjusted to pH 2 with H₃PO₄), + 1 mg/mL ascorbic acid | ≈ 60 | 230 | 85 | 37.0 | 99.3 |
| 3 | 2 mg **3b** in 1 mL DMSO | C | 15% EtOH, 85% 100 mM NaH₂PO₄ (adjusted to pH 2 with H₃PO₄) | ≈ 60 | 249 | 102 | 41.0 | 100 |
| 4 | 2 mg **3b** in 1 mL DMSO | C | 15% EtOH, 85% 100 mM NaH₂PO₄ (adjusted to pH 2 with H₃PO₄) | ≈ 60 | 236 | 91 | 38.6 | 99.7 |
| 5* | 2.4 mg **3b** in 1 mL DMSO | B | 23% MeCN,77% NaH₂PO₄/ Na₂HPO₄ buffer | ≈ 70 | 5.1 | 0.34 | 6.7 | 99.0 |
| 6 | 2.8 mg **3f** in 1 ml DMSO | C | 25% EtOH, 75% 50 mM NaOAc (adjusted to pH 2) | 55 | 1.0 | 0.18 | 17.8 | 100 |
| 7 | 3.1 mg **3e** in 1 mL DMSO | E | 25% EtOH, 75% 50 mM NaOAc (adjusted to pH 2) | 60 | 3.4 | 0.6 | 17.7 | 100 |
| 8 | 2 mg **3b** in 1 mL DMSO | C | 15% EtOH, 85% 100 mM NaH₂PO₄ (adjusted to pH 2.4 with H₃PO₄) | ≈ 60 | 58.8 | 17.3 | 29.5 | 99.1 |
| 9 | 2 mg **3b** | C | 15% EtOH, 85% 100 mM NaH₂PO₄ + 400 | ≈ 60 | 13.8 | 4.0 | 28.7 | 99.5 |
| | in 1 mL DMSO | | mM NaCl (adjusted to pH 2.4 with H₃PO₄) | | | | | |
| 10 | 2 mg **3f** in 1 mL DMSO | C | 15% EtOH, 85% 100 mM NaH₂PO₄ (adjusted to pH 2.4 with H₃PO₄) | ≈ 60 | 9.2 | 1.5 | 17 | 100 |
| 11 | 2 mg **3e** in 1 mL DMSO | F | 15% EtOH, 85% 100 mM NaH₂PO₄ (adjusted to pH 2.4 with H₃PO₄) | ≈ 65 | 48 | 11.5 | 23.9 | 100 |
| 12 | 2 mg **3e** in 1 mL MeCN | F | 15% EtOH, 85% 100 mM NaH₂PO₄ (adjusted to pH 2.4 with H₃PO₄) | ≈ 60 | 35.6 | 7 | 19.6 | 100 |
| 13 | 2 mg **3g** in 1 mL DMSO | C | 15% EtOH, 85% 100 mM NaH₂PO₄ (adjusted to pH 2.4 with H₃PO₄) | ≈ 60 | 1.1 | 0.066 | 5.9 | 99.6 |

| | | | | | | | | |
|---|---|---|---|---|---|---|---|---|
| * comparative examples | | | | | | | | |

## Claims

1. A method of preparing a compound of the formula I 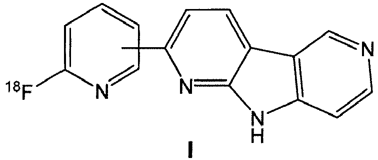 comprising the steps of
A) reacting a compound of the formula II with a ¹⁸F fluorinating agent 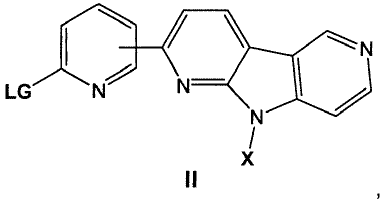 wherein **X** is H or **PG,**
**LG** is a leaving group, and
**PG** is an amine protecting group selected from carbobenzyloxy, (p-methoxybenzyl)oxycarbonyl, tert-butyloxycarbonyl, 9-fluorenylmethyloxycarbonyl, benzyl, p-methoxybenzyl, 3,4-dimethoxybenzyl, p-methoxyphenyl, triphenylmethyl, methoxyphenyl diphenylmethyl and dimethoxytrityl, and
B) optionally, if **X** is **PG,** cleaving the protecting group **PG,** and
C) subjecting the resultant compound of the formula **I** to high-performance liquid chromatography (HPLC) using a mobile phase comprising a mixture of ethanol and water.

2. The method according to claim 1, wherein X is PG, step B is absent and the protecting group PG is cleaved in step A or wherein X is PG and the protecting group PG is cleaved in step B.

3. The method according to claim 1 or 2, wherein the ratio of ethanol to water in the mobile phase is from 5/95 v/v to 80/20 v/v.

4. The method according to any of claims 1 to 3, wherein the pH of the mobile phase is from 0 to 8, preferably 1 to 3.

5. The method according to any of claims 1 to 4, wherein the mobile phase further comprises a buffer, which is preferably selected from alkali metal dihydrogen phosphates, di alkali metal hydrogen phosphates, tri alkali metal phosphates, alkali metal acetates, alkali metal formates, and mono/di/tri alkali metal citrates.

6. The method according to any one of claims 1 to 5, wherein the high-performance liquid chromatography (HPLC) is conducted under a pressure of 5*10⁶ to 4*10⁷ Pa (50 to 400 bars), preferably 5*10⁶ to 25*10⁶ Pa ( 50-250 bar).

7. The method according to any one of claims 1 to 6, wherein the high-performance liquid chromatography (HPLC) results in a fraction comprising the compound of the formula I and this fraction is subjected to a step D) sterile filtration.

8. The method according to any one of claims 1 to 7, wherein the method does not comprise solid phase extraction of the compound of the formula I after step C, preferably wherein the method does not comprise solid phase extraction of the compound of the formula I before or after step C.

9. The method according to any one of claims 1 to 8, wherein the compound of the formula I is not subjected to chromatography after the high-performance liquid chromatography (HPLC) of step C, preferably wherein the compound of the formula I is not subjected to chromatography other than the high-performance liquid chromatography (HPLC) of step C.

## Patentansprüche

1. Ein Verfahren zur Herstellung einer Verbindung der Formel I 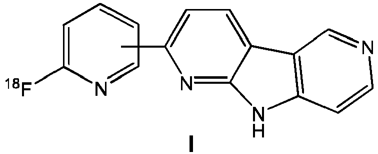 umfassend die Schritte des
A) Umsetzens einer Verbindung der Formel II mit einem ¹⁸F-Fluorierungsmittel 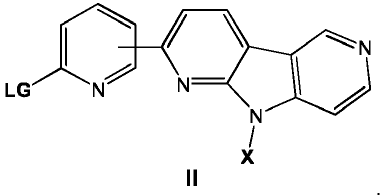 wobei X für H oder PG steht,
LG eine Abgangsgruppe ist, und
PG eine Aminschutzgruppe ist, ausgewählt aus Carbobenzyloxy, (p-Methoxybenzyl)oxycarbonyl, tert-Butyloxycarbonyl, 9-Fluorenylmethyloxycarbonyl, Benzyl, p-Methoxybenzyl, 3,4-Dimethoxybenzyl, p-Methoxyphenyl, Triphenylmethyl, Methoxyphenyldiphenylmethyl und Dimethoxytrityl, und
B) gegebenenfalls, wenn **X** gleich **PG** ist, Abspalten der Schutzgruppe **PG,** und
C) Unterziehen der resultierenden Verbindung der Formel **I** einer Hochleistungs-Flüssigkeitschromatographie (HPLC) unter Verwendung einer mobilen Phase, umfassend ein Gemisch von Ethanol und Wasser.

2. Das Verfahren gemäß Anspruch 1, wobei X gleich PG ist, Schritt B fehlt und die Schutzgruppe PG in Schritt A abgespalten wird, oder wobei X gleich PG ist und die Schutzgruppe PG in Schritt B abgespalten wird.

3. Das Verfahren gemäß Anspruch 1 oder 2, wobei das Verhältnis von Ethanol zu Wasser in der mobilen Phase 5/95 v/v bis 80/20 v/v beträgt.

4. Das Verfahren gemäß einem der Ansprüche 1 bis 3, wobei der pH-Wert der mobilen Phase von 0 bis 8, vorzugsweise von 1 bis 3 beträgt.

5. Das Verfahren gemäß einem der Ansprüche 1 bis 4, wobei die mobile Phase ferner einen Puffer umfasst, welcher bevorzugt ausgewählt ist aus Alkalimetalldihydrogenphosphaten, Dialkalimetallhydrogenphosphaten, Trialkalimetallphosphaten, Alkalimetallacetaten, Alkalimetallformiaten und Mono/Di/Tri-Alkalimetallcitraten.

6. Das Verfahren gemäß einem der Ansprüche 1 bis 5, wobei the Hochleistungs-Flüssigkeitschromatographie (HPLC) unter einem Druck von 5*10⁶ bis 4*10⁷ Pa (50 bis 400 bar), vorzugsweise 5*10⁶ bis 25*10⁶ Pa (50 bis 250 bar) durchgeführt wird.

7. Das Verfahren gemäß einem der Ansprüche 1 bis 6, wobei die Hochleistungs-Flüssigkeitschromatographie (HPLC) zu einer Fraktion, umfassend die Verbindung der Formel I, führt und diese Fraktion einem Schritt D), steriler Filtration, unterzogen wird.

8. Das Verfahren gemäß einem der Ansprüche 1 bis 7, wobei das Verfahren keine Festphasenextraktion der Verbindung der Formel I nach Schritt C umfasst, vorzugsweise, wobei das Verfahren keine Festphasenextraktion der Verbindung der Formel I vor oder nach Schritt C umfasst.

9. Das Verfahren gemäß einem der Ansprüche 1 bis 8, wobei die Verbindung der Formel I nach der Hochleistungs-Flüssigkeitschromatographie (HPLC) des Schritts C keiner Chromatographie unterzogen wird, vorzugsweise wobei die Verbindung der Formel I keiner Chromatographie außer der Hochleistungs-Flüssigkeitschromatographie (HPLC) des Schritts C unterzogen wird.

## Revendications

1. Méthode de préparation d'un composé de formule I 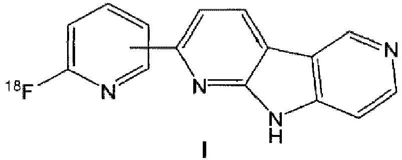 comprenant les étapes de
A) réaction d'un composé de formule II avec un agent de fluoration au ¹⁸F 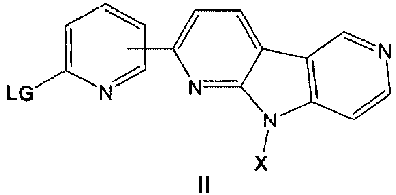 dans laquelle X est H ou PG,
LG est un groupe partant, et
PG est un groupe protecteur d'amine choisi parmi carbobenzyloxy, (p-méthoxybenzyl)oxycarbonyle, tert-butyloxycarbonyle, 9-fluorénylméthyloxycarbonyle, benzyle, p-méthoxybenzyle, 3,4-diméthoxybenzyle, p-méthoxyphényle, triphénylméthyle, méthoxyphényldiphénylméthyle et diméthoxytrityle, et
B) éventuellement, si X est PG, clivage du groupe protecteur PG, et
C) soumission du composé résultant de formule I à une chromatographie liquide haute performance (CLHP) utilisant une phase mobile comprenant un mélange d'éthanol et d'eau.

2. Méthode selon la revendication 1, dans laquelle X est PG, l'étape B est absente, et le groupe protecteur PG est clivé dans l'étape A, ou dans laquelle X est PG et le groupe protecteur PG est clivé dans l'étape B.

3. Méthode selon la revendication 1 ou 2, dans laquelle le ratio entre l'éthanol et l'eau dans la phase mobile est de 5/95 v/v à 80/20 v/v.

4. Méthode selon l'une quelconque des revendications 1 à 3, dans laquelle le pH de la phase mobile est de 0 à 8, de préférence de 1 à 3.

5. Méthode selon l'une quelconque des revendications 1 à 4, dans laquelle la phase mobile comprend en outre un tampon, qui est de préférence choisi parmi les dihydrogénophosphates de métal alcalin, les hydrogénophosphates de di(métal alcalin), les phosphates de tri(métal alcalin), les acétates de métal alcalin, les formiates de métal alcalin, et les citrates de mono/di/tri(métal alcalin).

6. Méthode selon l'une quelconque des revendications 1 à 5, dans laquelle la chromatographie liquide haute performance (CLHP) est effectuée sous une pression de 5^{∗}10⁶ à 4^{∗}10⁷ Pa (50 à 400 bars), de préférence de 5^{∗}10⁶ à 25^{∗}10⁶ Pa (50 à 250 bars).

7. Méthode selon l'une quelconque des revendications 1 à 6, dans laquelle la chromatographie liquide haute performance (CLHP) a pour résultat une fraction comprenant le composé de formule I, et cette fraction est soumise à une étape D) de filtration stérile.

8. Méthode selon l'une quelconque des revendications 1 à 7, laquelle méthode ne comprend pas d'extraction en phase solide du composé de formule I après l'étape C, de préférence laquelle méthode ne comprend pas d'extraction en phase solide du composé de formule I avant ou après l'étape C.

9. Méthode selon l'une quelconque des revendications 1 à 8, dans laquelle le composé de formule I n'est pas soumis à une chromatographie après la chromatographie liquide haute performance (CLHP) de l'étape C, de préférence dans laquelle le composé de formule I n'est pas soumis à une chromatographie autre que la chromatographie liquide haute performance (CLHP) de l'étape C.
